# EUROPEAN PATENT SPECIFICATION

(11) **EP 2 534 488 B1**
(45) Date of publication and mention of the grant of the patent: **01.10.2014**
(21) Application number: 11707106.8
(22) Date of filing: 10.02.2011
(51) Int. Cl.: G01N 33/92, G01N 33/574

(54) **PHOSPHOLIPID PROFILING OF CANCER**
PHOSPHOLIPIDPROFILIERUNG VON KREBS
ÉTABLISSEMENT DU PROFIL DE PHOSPHOLIPIDES D'UN CANCER

(30) Priority: 09.03.2010 GB 201003814; 11.02.2010 GB 201002366
(43) Date of publication of application: 19.12.2012
(73) Proprietor: Katholieke Universiteit Leuven, 3000 Leuven (BE)
(72) Inventor: SWINNEN, Johan, B-3000 Leuven (BE)
(74) Representative: Laenen, Bart Roger Albert
(86) International application number: PCT/EP2011/051936
(87) International publication number: WO 2011/098509

(56) References cited:
- SWINNEN JOHANNES V ET AL: "Increased lipogenesis in cancer cells: new players, novel targets", CURRENT OPINION IN CLINICAL NUTRITION AND METABOLIC CARE, RAPID SCIENCE PUBLISHERS, LONDON, GB, vol. 9, no. 4, 1 July 2006 (2006-07-01), pages 358-365, XP009148976, ISSN: 1363-1950
- SHIMMA ET AL: "MALDI-based imaging mass spectrometry revealed abnormal distribution of phospholipids in colon cancer liver metastasis", JOURNAL OF CHROMATOGRAPHY B: BIOMEDICAL SCIENCES & APPLICATIONS, ELSEVIER, AMSTERDAM, NL, vol. 855, no. 1, 20 July 2007 (2007-07-20) , pages 98-103, XP022162171, ISSN: 1570-0232, DOI: DOI:10.1016/J.JCHROMB.2007.02.037
- BISWAS S K ET AL: "LIPID COMPOSITION FLUIDITY AND LECTIN-REACTIVITY OF MEMBRANES FROM NORMAL HUMAN UTERUS AND BENIGN AND MALIGNANT UTERINE TUMORS", INDIAN JOURNAL OF BIOCHEMISTRY AND BIOPHYSICS, COUNCIL OF SCIENTIFIC & INDUSTRIAL RESEARCH, NEW DEHLI, IN, vol. 23, no. 1, 1 January 1986 (1986-01-01), pages 13-18, XP009149059, ISSN: 0301-1208
- MENENDEZ JAVIER A ET AL: "Fatty acid synthase and the lipogenic phenotype in cancer pathogenesis", NATURE REVIEWS CANCER, vol. 7, no. 10, October 2007 (2007-10), pages 763-777, XP002640983, ISSN: 1474-175X
- SWINNEN J V: "Increased lipogenesis in steroid-responsive cancer cells: mechanisms of regulation, role in cancer cell biology and perspectives on clinical applications", VERHANDELINGEN - KONINKLIJKE ACADEMIE VOOR GENEESKUNDE VAN BELGIE, ACADEMIE VOOR GENEESKUNDE VAN BELGIE, BRUSSEL, BE, vol. 63, no. 4, 1 January 2001 (2001-01-01), pages 321-333, XP009148977, ISSN: 0302-6469
- BRUSSELMANS KOEN ET AL: "The Lipogenic Switch in Cancer", MITOCHONDRIA AND CANCER, SPRINGER, NEW YORK, US, 1 January 2009 (2009-01-01), pages 39-59, XP009149061, DOI: DOI:10.1007/978-0-387-84834-1_3 ISBN: 978-0-387-84834-1
- MILNE S ET AL: "Lipidomics: An analysis of cellular lipids by ESI-MS", METHODS : A COMPANION TO METHODS IN ENZYMOLOGY, ACADEMIC PRESS INC., NEW YORK, NY, US, vol. 39, no. 2, 1 June 2006 (2006-06-01), pages 92-103, XP024908534, ISSN: 1046-2023, DOI: DOI:10.1016/J.YMETH.2006.05.014 [retrieved on 2006-06-01]
- RYSMAN EVELIEN ET AL: "De novo lipogenesis protects cancer cells from free radicals and chemotherapeutics by promoting membrane lipid saturation", CANCER RESEARCH, AMERICAN ASSOCIATION FOR CANCER REREARCH, US, vol. 70, no. 20, 1 October 2010 (2010-10-01), pages 8117-8126, XP009148979, ISSN: 0008-5472

## Description

### TECHNICAL FIELD

In general, the present invention provides prognostic and predictive methods and kits for determining the lipogenicity of a tumor in a subject, by making use of phospholipid profiling, whereby a relative increase in mono-unsaturated phospholipids species in combination with a relative decrease in poly-unsaturated phospholipid species is indicative for a more resistant and aggressive lipogenic cancer phenotype.

### BACKGROUND TO THE INVENTION

Cancer is recognized by uncontrolled growth of cells, which form a tumor and ultimately may invade other tissues (metastasis). Cancer affects people of all ages, and is a major cause of human death whereby in particular lung cancer, stomach cancer, colorectal cancer and liver cancer are the most deadliest ones. The most commonly occurring cancer in men is prostate cancer and in women it is breast cancer.

Each year 11 million people worldwide are diagnosed with cancer. Almost 7 million die from the disease. Treatment options of many cancer types and the success rate of intervention largely depend on the stage of the disease at the time of diagnosis. In many cases, early detection is of utmost importance as it greatly increases the chances for successful treatment. The implementation of screening programs for early detection, however, increases the detection rate of latent and clinically irrelevant tumors. This poses a great risk of overtreatment and is therefore a tremendous clinical dilemma since no effective tests for disease progression are available. On the other hand, the biochemical failure is significant notwithstanding locally aggressive therapy. A substantial number of patients will develop clinical metastatic disease or already presents with occult metastases at the time of staging. Together with the fact that tumors may respond differently to a given type of therapy, these situations emphasize the need for reliable criteria for treatment decision.

Currently, treatment decisions of many cancers are largely based on the histopathological TNM (tumor, node, metastasis) system and the histological differentiation of the primary tumor. As these systems provide only an *ad hoc* picture of the tumor, molecular-based insights in the tumor characteristics will undoubtedly help to more accurately predict progression and therapy response and will aid in the selection of more appropriate primary and/or adjuvant treatment options. Recent advances in molecular analytical methods hold significant promise in this respect and have the power to revolutionize the ways cancer is diagnosed and treated.

Up to now, most efforts towards molecular characterization and staging of cancer are carried out at the level of DNA or RNA (screening for genomic mutations, analysis of epigenetics, or transcriptome analysis). For example, gene expression profiling can predict clinical outcomes of prostate cancer (G.V. et al., J. CHn. Invest. 113: 913-923 (2004)) as well as breast cancer (Van 't Veer et al. Nature 415: 530-536 (2002)). Furthermore, Glinsky et al. (J. Clin. Invest. [upsilon]5: 1503-1521 (2005)) teaches that altered expression of the BMI1 oncogene is linked with a poor prognosis profile in patients with multiple types of cancer. However, use of these predictive markers is mostly limited to one or only a few types of cancer, and not generally applicable to all types of cancer. Therefore, changes more downstream are perhaps of more significance as they represent more distal endpoints of cellular regulation, integrating diverse (epi)genetic, regulatory and environmental cues. Of particular interest in this respect are changes in membrane lipid composition.

Functioning as barriers that separate and compartmentalize the cell's content, membranes function as unique interfaces at which numerous cellular processes (including signaling, nutrient transport, cell division, respiration, cell death mechanisms, etc) are concentrated and regulated. An ever increasing body of evidence indicates that membrane lipids, and particularly changes in phospholipid species play a central role in this regulation (Marguet et al., 2006).

Phospholipids are a complex class of cellular lipids that are composed of a headgroup (choline, ethanolamine, serine, inositol, etc) and 1 to 4 fatty acyl chains that can differ both in length and in the number of unsaturations (double bonds), leading to hundreds of different species. The building blocks for these lipids can be taken up from the circulation, however, some can also be synthesized *de novo.*

Most cells express elaborate pathways that dynamically modify lipid structures. This can change their chemical properties dramatically and locally modulate the biochemical and biophysical properties of membranes.

For example Shimma et al., 2007 describes the identification of abnormal distribution of phospholipids in colon cancer metastasis, especially phosphatidylcholine and sphingomyelin, by MALDI imaging (Shimma et al., 2007; Journal of Chrom. B: Biomedical Sciences & Applications Vol. 855, no 1 - pp. 98-103). Biswas et al., 1986 discloses fatty acid compositions which are determined in different tumors and normal tissue as well as the saturation and unsaturation of said fatty acids (Biswas et al., 1986 - Indian Journal of Biochemistry & Biophysics Vol. 23 - pp 13-18). Rysman et al. 2010 teaches that the lipogenic phenotype of cancer cells comprises enrichment of saturated and mono-unsaturated acyl chains and no increase of polyunsaturated acyl chains (Rysman et al., 2010; Cancer Research - Vol. 70, no. 20 - pp 8117-8126).

There is mounting evidence that in tumors, phospholipid metabolism is dramatically different from normal tissue. Whereas most normal tissues acquire the bulk of the required lipids from the circulation, tumor cells frequently synthesize the majority of their lipids *de novo* (Kuhajada, 2006; Swinnen et al., 2006; Menendez et al., 2008; Brusselmans et al., 2009). This is illustrated by a dramatic overexpression of lipogenic enzymes such as fatty acid synthase in some tumors, particularly in those with a poor prognosis. For example, Swinnen et al. 2001 shows that FAS may be used as a prognostic marker for aggressive disease (Swinnen et al., 2001 Koninklijke Academie voor Geneeskunde van België vol. 63, no. 4, pp 321-333). Inhibition of this pathway attenuates tumor growth, kills cancer cells and makes them more responsive to various cancer treatments, indicating that the lipogenic phenotype contributes to cancer progression and that lipogenicity of a tumor is a marker for aggressive and poorly responsive tumors. However, since activation of this lipogenic pathway involves changes at multiple levels of enzyme regulation (genetic changes, enhanced transcription and translation, protein stabilization and phosphorylation, allosteric regulation and substrate flux), there are currently no reliable markers for identifying lipogenic tumors in general. Furthermore, activation of said lipogenic pathway occurs downstream of various common oncogenic events (loss of PTEN, activation of Akt, loss of BRCA1, steroid hormone action, tumor-associated hypoxia, etc.) (Kuhajada, 2006; Swinnen et al., 2006), and therefore, said common cancer markers such as PTEN, Akt, BRCA1,... are also not useful to determine whether a tumor is lipogenic or not. As such there is a need for good predictive markers to determine the lipogenic phenotype of a tumor. Said markers are then useful for predicting the evolution of tumors in general, as well as for analyzing the effect of cancer therapies.

We have now found that lipogenic tumors have significantly different phospholipid profiles compared to non-lipogenic tumors. Therefore, by making use of phospholipid profiling, lipogenic tumors can be easily distinguished from non-lipogenic tumors, making it much easier to fine-tune cancer therapy for each individual patient.

Correlations between dietary fatty acids and the risk of developing cancer have been described previously. For example, increased intake of particular n-6 polyunsaturated fatty acids (linoleic acid; Godley et al., 1996 and Gann et al., 1994), saturated fatty acids (palmitic acid; Harvei et al., 1997)(myristic acid; Männistö et al., 2003), and monosaturated fatty acids (palmitoleic acid; Harvei et al., 1997) have shown association with an increased risk of developing cancer. Furthermore, dietary fatty acids have also been shown to correlate with the aggressiveness of tumors. For example Crowe et al. showed that palmitic acid (saturated fatty acid) was positively correlated with low-grade (less aggressive) prostate cancer, whereas myristic acid (saturated fatty acid) as well as linolenic acid and eicosapentaenoic acid (both n-3 polyunsaturated fatty acids) were positively correlated with high-grade (aggressive) prostate cancer (Crowe et al., 2008). However, since lipogenic tumors synthesize most of their phospholipids *de novo,* levels of dietary fatty acids are mostly not useful for analyzing the lipogenicity of a tumor.

Furthermore, although some inelaborate phospholipids profiles have been found to be associated with the tumorigenicity of a tumor, no correlations with the lipogenicity of said tumors have been described so far. For example, Le Bivic et al. (1987) showed that the ratio of monounsaturated-to-polyunsaturated phospholipids is increased in highly tumorigenic human melanoma cell lines. Similar results were obtained in microsomes and microchondria of diethylnitrosamine (DEN)-induced hepatomas, which showed an increased ratio of monounsaturated-to-polyunsaturated PC and PE fatty acids compared to those found in normal liver cells (Canuto et al., 1989). Both authors used a similar technique for isolating and analyzing the individual lipid species. Total lipids were first extracted in chloroform-methanol, and the different classes of phospholipids were then separated by thin-layered chromatography (TLC). The phospholipids fractions were then treated to obtain fatty acid methyl esters which were further analyzed by gas-liquid chromatography (GLC). Although GLC is useful for giving a general idea of the fatty acid composition of cells, only a limited number of distinct fatty acids can be detected herewith, resulting in inelaborate phospholipids profiles. In as far these limited profiles have been linked to tumorigenicity, none of these studies hints or suggests to the use a possible association of these limited phospholipids profiles with the lipogenicity of a tumor.

We have now found that phospholipid profiles are useful for predicting the lipogenicity of a tumor, in general for all types of tumors. Furthermore, it allows to fine-tune cancer therapy for each individual patient, as well as for analyzing the effects of cancer therapy.

In particular, we found that a general increase in mono-unsaturated phospholipid species and an overall decrease in polyunsaturated phospholipid species is correlated with increased FASN expression and provides tumor cells with increased resistance against apoptosis, stress radicals and chemotherapy. These findings indicate that membrane phospholipid profiling can be used for the development of prognostic as well as predictive tests for assessing the lipogenicity of a tumor in a subject.

In particular, a general increase in mono-unsaturated phosphatidylcholine species and an overall decrease in polyunsaturated phosphaditylcholine species is correlated with increased FASN expression and provides tumor cells with increased resistance against apoptosis, stress radicals and chemotherapy.

Implementation of the use of such phospholipid profiles as predictive/prognostic biomarkers will lead to a more personalized medicine in which diagnosis and treatment are more interdependent and based on molecular evidence of how a tumor will evolve and respond to a particular treatment. These advances will allow the physician to tailor the treatment to the patient's individual needs. It will also avoid excess of morbidity and side effects due to overtreatment (i.e. sparing the patient from an invasive surgical procedure in case of nonaggressive or too advanced disease), and will optimize the use of available resources.

### SUMMARY OF THE INVENTION

An objective of the present invention was to provide a prognostic or predictive *in vitro* method for determining the lipogenicity of a tumor in a subject, said method comprising; determining the relative expression level of at least 1 mono-unsaturated phospholipid; and at least 1 poly-unsaturated phospholipid, in tumor sample versus normal sample; wherein an increase in relative expression level of said mono-unsaturated phospholipids and a decrease in relative expression level of said poly-unsaturated phospholipids is indicative for the more aggressive lipogenic phenotype.

In a further embodiment, the *in vitro* method according to the present invention comprises; determining the expression level of at least 1 mono-unsaturated phospholipid; and at least 1 poly-unsaturated phospholipid, in tumor sample and normal sample; wherein an increased ratio of mono-unsaturated versus poly-unsaturated phospholipids in the tumor sample compared to the normal sample is indicative for a more aggressive lipogenic phenotype.

In a particular embodiment, the in vitro method according to the present invention comprises; determining the lipogenic profile of a tumor in a patient; wherein an increase in species with one or two unsaturations (in both acyl chains together), and a decrease in PL species with more than 3 unsaturations is indicative for a more aggressive lipogenic phenotype, in particular said PL species are phosphatidylcholine species.

In a further embodiment, the phospholipids are selected from the group comprising; glycerophospholipid, phosphatidic acid, phosphatidylethanolamine, phosphatidylcholine, phosphatidylserine and phosphoinositides, preferably phosphatidylcholine.

In a further embodiment, the saturated phospholipids are the saturated phosphatidylcholine species, selected from the group comprising PC30:0, PC32:0, PC34:0, PC36:0, and PC38:0; and/or the saturated phosphatidylethanolamines, selected from the group comprising PE36:0 and PE38:0; and/or the saturated phosphatidylserines selected from the group comprising PS36:0, PS38:0, PS40:0 and PS42:0; and/or the saturated phosphatidylinositides selected from the group comprising PI34:0, PI36:0 and PI38:0.

In yet a further embodiment, the mono-unsaturated phospholipids are the phosphatidylcholines (PC) with one or two mono-unsaturated fatty acyl chains, selected from the group comprising PC30:1, PC30:2, PC32:1, PC32:2, PC34:1, PC34:2, PC36:1, PC36:2, PC38:1 and PC38:2; and/or the phosphatidylethanolamines (PE) with one or two mono-unsaturated fatty acyl chains, selected from the group comprising PE32:1 and PE34:2; and/or the phosphatidylserines (PS) with one or two mono-unsaturated fatty acyl chains, selected from the group comprising PS36:2, PS38:2, PS40:2, and PS42:2; and/or the phosphatidylinositides (PI) with one or two mono-unsaturated fatty acyl chains, selected from the group comprising PI34:1, PI36:1, PI38:1, PI34:2, PI36:2 and PI38:2.

In a further embodiment, the poly-unsaturated phospholipids are poly-unsaturated phosphatidylcholines (PC), selected from the group comprising PC34:3, PC34:4, PC36:3, PC36:4, PC36:5, PC38:3, PC38:4, PC38:5, PC38:6, PC40:3, PC40:4, PC40:5, PC40:6 and PC40:7; and/or poly-unsaturated phosphatidylethanolamines (PE), selected from the group comprising PE36:4, PE38:4 and PE40:4; and/or poly-unsaturated phosphatidylserines (PS), selected from the group comprising PS38:4, PS40:4, PS38:5, PS40:5 and PS38:6; and/or poly-unsaturated phosphatidylinositides (PI), selected from the group comprising; PI36:4 and PI38:4.

In a preferred embodiment, the mono-unsaturated phospholipids are the mono-unsaturated phosphatidylcholines (PC) PC34:1; and the poly-unsaturated phospholipids are the poly-unsaturated phosphatidylcholines (PC), selected from the group comprising PC36:3, PC38:3, PC36:4, PC38:4, PC40:4, PC36:5, PC38:5, PC40:5.

In yet another preferred embodiment the mono-unsaturated phospholipids are the mono-unsaturated phosphatidylcholines (PC) PC34:1; and the poly-unsaturated phospholipids are the poly-unsaturated phosphatidylcholines (PC) PC36:4 or PC38:4.

In a further embodiment, in addition to determining the phospholipid profiles, the relative expression level of one or more other biomarkers for an aggressive lipogenic phenotype may be determined, such as for example, but not limited to, FASN (fatty acid synthase), ACCA (acetyl CoA carboxylase alpha), choline kinase and ACLY (ATP citrate lyase) expression. A tumor having an aggressive lipogenic phenotype may than for example be identified by an increase in relative expression level of mono-unsaturated phospholipids, a decrease in relative expression level of poly-unsaturated phospholipids, and an increase in expression or phosphorylation/activation of one or more other biomarker for an aggressive lipogenic phenotype. Alternatively, a tumor having an aggressive lipogenic phenotype may for example be identified by an increased ratio of mono-unsaturated versus poly-unsaturated phospholipids in the tumor sample compared to the normal sample, and an increase in expression of one or more other biomarker for an aggressive lipogenic phenotype.

The invention further relates to the use of the *in vitro* method according to the present invention for determining the lipogenicity of a tumor in a subject.

Finally, the invention provides a kit for performing the *in vitro* method according to this invention, said kit comprising; the reagents for the ESI - MS/MS sample preparation of intact phospholipids, in particular an antioxidant, solvents and standards.

### BRIEF DESCRIPTION OF THE DRAWINGS

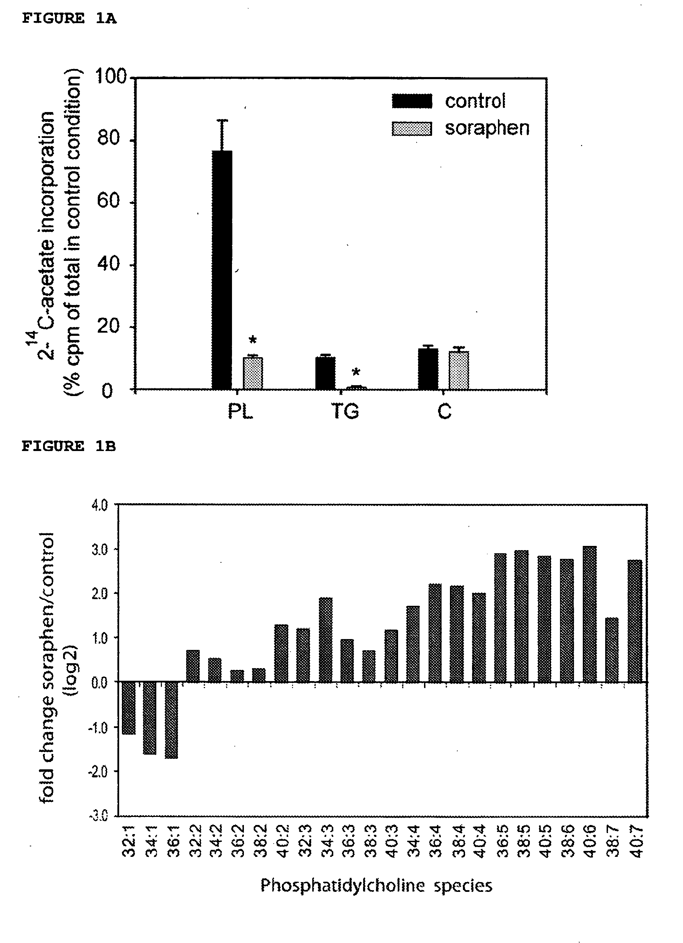

**Figure 1****.** Impact of soraphen treatment on intact phosphatidylcholine species. LNCaP cells were treated with soraphen (100 nM) or vehicle (control) for 72 hr. Lipid extracts were prepared, and the phosphatidylcholine species were analyzed by ESI-MS/MS in the MRM mode. The m/z of major species and their species assignment are indicated. Std refers to the lipid standards. Lipid profiling was performed in three pairs of samples (1-3), and expressed as the average thereof. Changes are expressed as fold-change (log 2) in soraphen treated versus control samples for the individual lipid species.

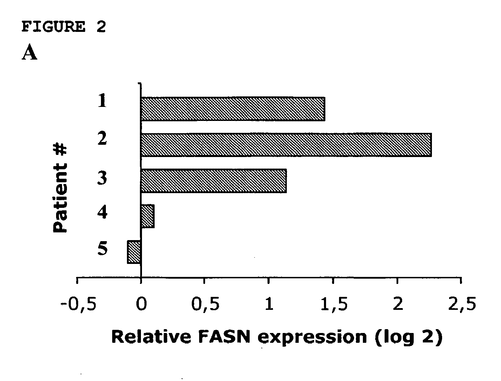

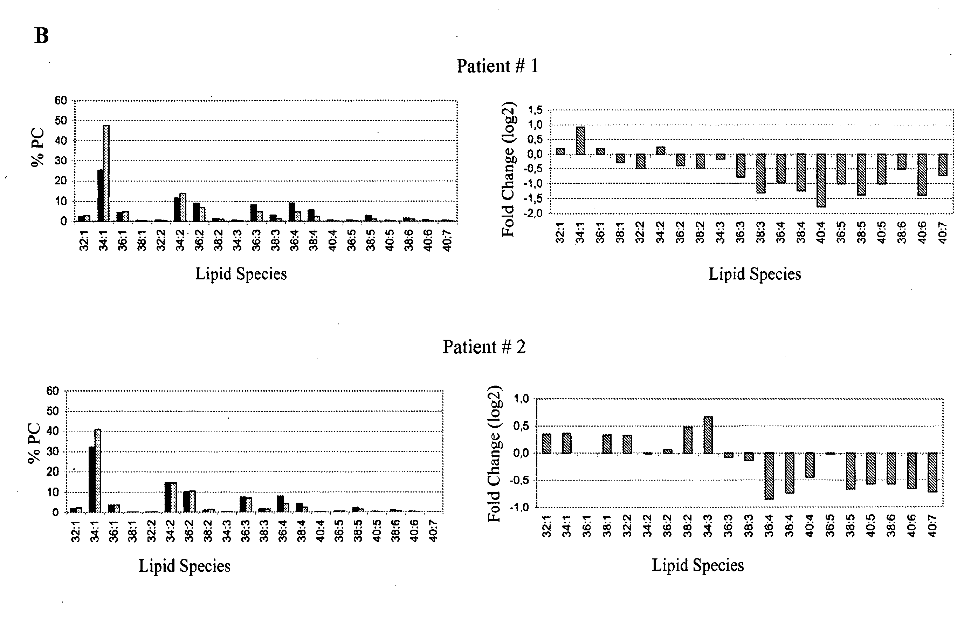

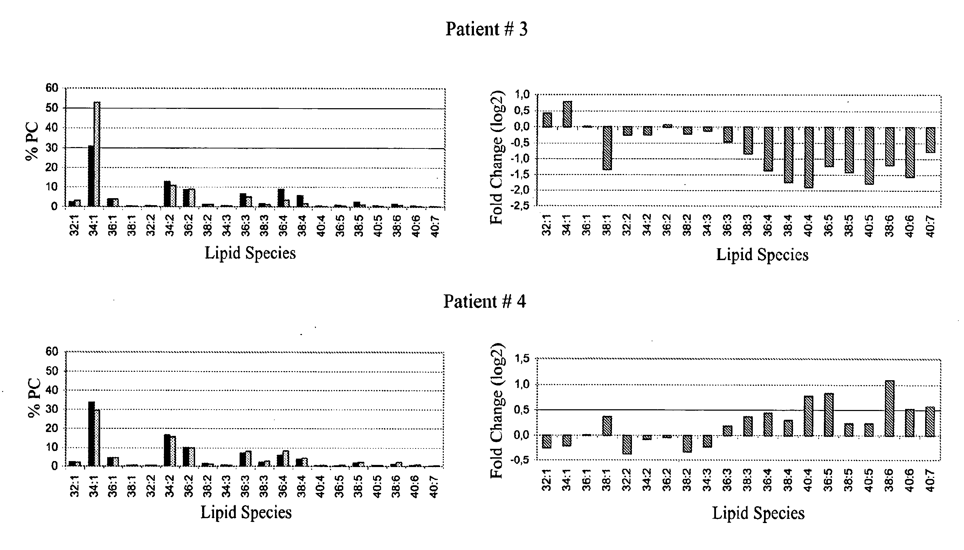

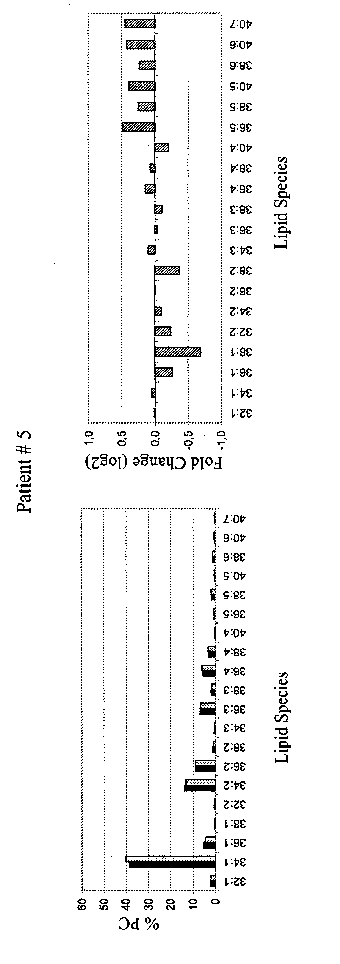

**Figure 2****. A.** Expression of fatty acid synthase (FASN) in five matched pairs of malignant versus normal prostate tissue specimens was measured by western blotting analysis, and the expression was normalized to β-actin expression. Values are expressed as a ratio of tumor over matching normal tissue. **B.** The relative abundances of PC species in the five matched pairs of malignant versus normal tissues were recorded by ESI-MS/MS in the MRM mode. The results are in the left panels expressed as % of individual lipid species compared to total phoshatidylcholine levels in normal (black bars) and malignant (grey bars) samples; and in the right panels as fold-change (log 2) in normal versus malignant sample for the individual lipid species.

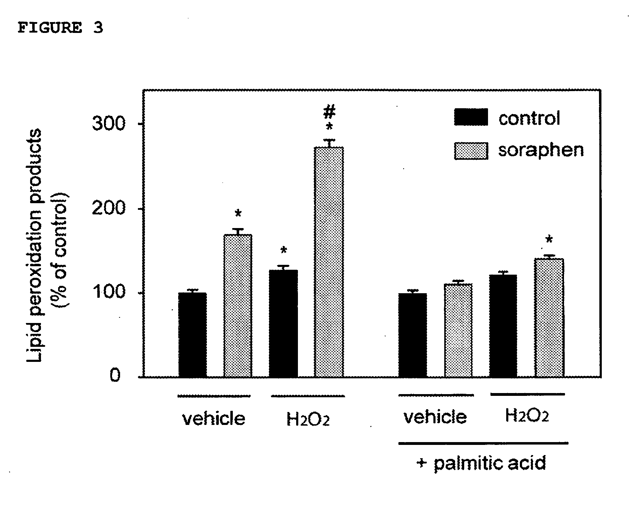

**Figure 3****.** (A) Effects of soraphen, H₂O₂, and palmitic acid on lipid peroxidation products. LNCaP cells were treated with soraphen (100 nM) or vehicle (control) in the presence or absence of exogenous palmitic acid (75 µM) for 72 hr. During the last 2 hr, the cells were exposed to 200 µM H₂O₂. Equal amounts of cells were analyzed for lipid peroxidation products using a lipid peroxidation assay kit. Data represent mean ± SE (n=4). *Significantly different (p<0.05) from control without H₂O₂ exposure. #Significantly different (p<0.05) from either treatment with H₂O₂ or soraphen alone.

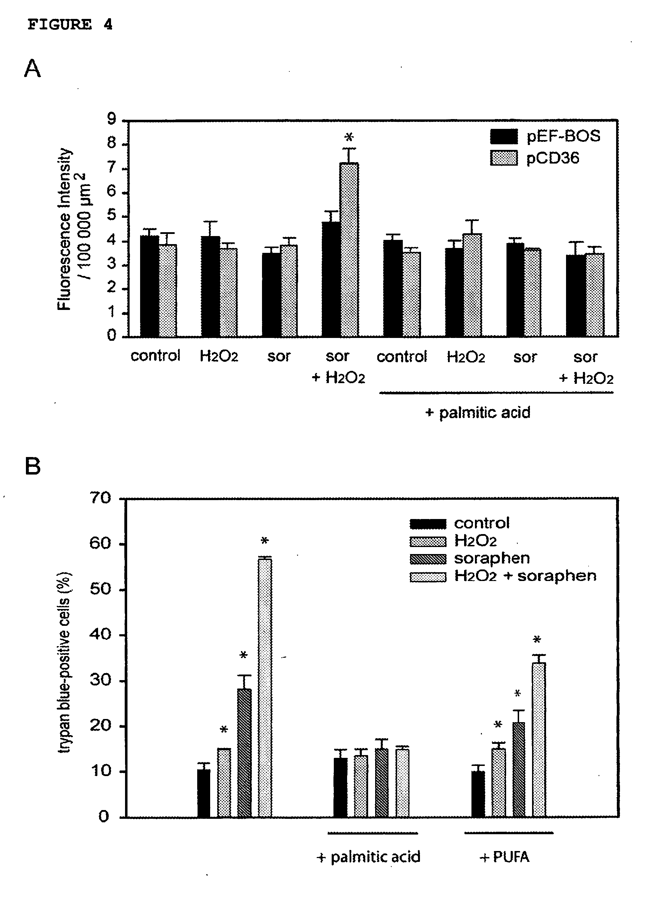

**Figure 4****.** (A) Impact of *de novo* lipogenesis on recognition by CD36-positive cells. LNCaP cells were treated with soraphen (100 nM) or vehicle (control) in the presence or absence of exogenous palmitic acid (75 µM) for 72 hr. During the last 30 min, the cells were exposed to 300 µM H₂O₂. Cells were trypsinized, labeled with Cell Tracker Orange CMRA, and plated on top of COS-7 cells that had been transfected with a plasmid encoding CD36 (pCD36) or the corresponding empty vector (pEF-BOS). After 1 hr, the cultures were extensively washed and imaged. The fluorescence was quantified using Adobe Photoshop software. Data represent means ± SE (n=3). *Significantly different (p<0.05) from control. (B) Effect of *de novo* lipogenesis on the cell's sensitivity to oxidative stress-induced cell death. LNCaP cells were treated with soraphen (100 nM) or vehicle (control) in the presence or absence of exogenous palmitic acid (75 pM) or with a mixture of 10 % palmitic, 45 % linoleic and 45 % linolenic acid (PUFA) (75 µM total) for 72 hr. During the last 24 hr, cells were exposed to 300 µM H₂O₂. Cells were collected and stained with trypan blue to assess the cell viability. Data represent means ± SE (n=3). *Significantly different (p<0.05) from control.

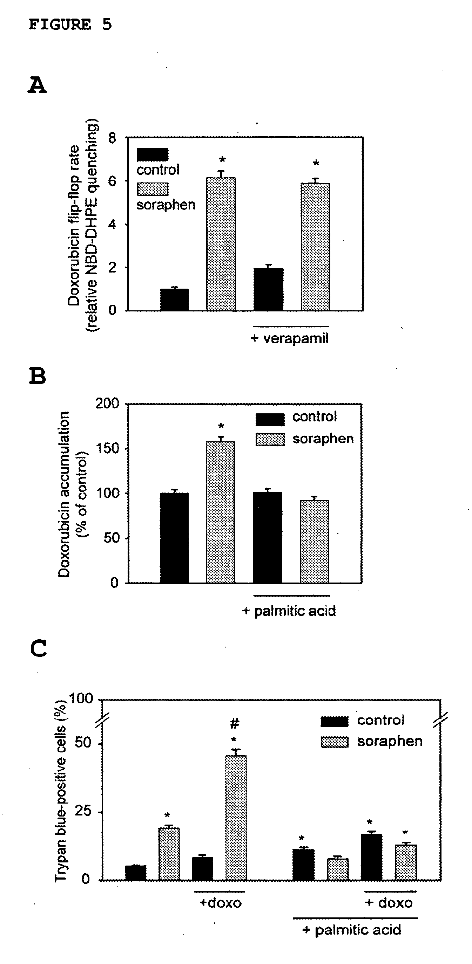

**Figure 5****.** (A) Impact of soraphen on the flip-flop rate of doxorubicin. LNCaP cells were cultured with or without soraphen (100 nM) for 72 hr. During the last 24 hr, NBD-DHPE (10 µM) was added to the cells. An hour prior to analysis, the cells were treated with vehicle or verapamil (100 µM). Equal amounts of cells were collected, doxorubicin (10 µM) was added, and the NBD fluorescence was continuously monitored to determine the level of quenching of NBD by doxorubicin. Data represent means ± SE (n=4). *Significantly different (p<0.05) from control. (B) Quantification of the effect of soraphen on doxorubicin accumulation in LNCaP cells. LNCaP cells were treated with or without soraphen (100 nM) for 72 hr in the presence or absence of palmitic acid (75 µM). Thirty minutes prior to analysis, 10 µM of doxorubicin was added to the cells. Cellular extracts were prepared and doxorubicin content was fluorimetrically determined. Data represent means ± SE (n=4). *Significantly different (p<0.05) from control. (C) Effect of soraphen and palmitic acid on doxorubicin-induced cytotoxicity in LNCaP cells. LNCaP cells were treated with or without soraphen (100 nM) for 72 hr in the presence or absence of palmitic acid (75 µM). During the last 24 hr, doxorubicin (4 µM) or vehicle was added to the cells. The cells were collected and stained with trypan blue to assess the cell viability. Data represent means ± SE (n=6-12). *Significantly different (p<0.05) from control. ^{#}Significantly different (p<0.05) from either soraphen or doxorubicin alone.

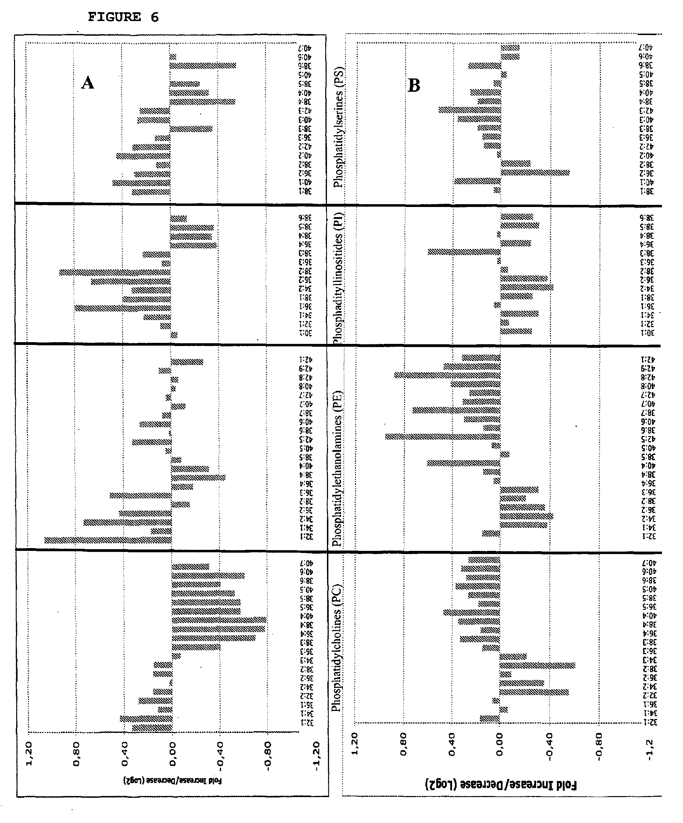

**Figure 6****.** Average of relative changes (log2) in phospholipid species in prostate tumor versus normal prostate tissue from 13 prostate cancer patients. Phospholipids are ordered according to lipid class (PC, PE, PS and PI). Cluster analysis divided the patients in 2 major groups. Cluster A: 9 patients with lipogenic phenotype (fig. 6A); Cluster B: 4 patients without lipogenic phenotype (fig. 6B).

### DESCRIPTION OF THE INVENTION

An objective of the present invention was to provide an *in vitro* method for determining the lipogenicity of a tumor in a subject, said method comprising; determining the relative expression level of at least 1 mono-unsaturated phospholipid; and at least 1 poly-unsaturated phospholipid, in tumor sample versus normal; wherein an increase in relative expression level of said mono-unsaturated phospholipids and a decrease in relative expression level of said poly-unsaturated phospholipids is indicative for a more aggressive lipogenic phenotype.

In a further embodiment, the in vitro method according to the invention further comprises determining the relative expression level of at least 1 saturated phospholipids; and wherein a decrease in relative expression level of said saturated phospholipids, an increase in relative expression level of said mono-unsaturated phospholipids and a decrease in relative expression level of said poly-unsaturated phospholipids is indicative for a more aggressive lipogenic phenotype

A further objective of the present invention was to provide an *in vitro* method for determining the lipogenicity of a tumor in a subject, said method comprising; determining the expression level of at least 1 mono-unsaturated phospholipid; and at least 1 poly-unsaturated phospholipid, in tumor sample and normal sample; and wherein an increased ratio of mono-unsaturated versus poly-unsaturated phospholipids in tumor sample compared to normal sample is indicative for a more aggressive lipogenic phenotype.

In a further embodiment the method of the present invention comprises determining the relative expression level of at least 2, 3, 4, 5, 6, 7, 8, 9, 10, 11, 12, 13, 14, 15, 16, 17, 18, 19 or 20 mono-unsaturated phospholipids.

In yet a further embodiment the method of the present invention comprises determining the relative expression level of at least 2, 3, 4, 5, 6, 7, 8, 9, 10, 11, 12, 13, 14, 15, 16, 17, 18, 19 or 20 poly-unsaturated phospholipids.

As used herein a tumor is defined as an abnormal growing mass of cells. Tumors useful for the present invention include but are not limited to prostate cancer, breast cancer, lung, colon, stomach, ovaries, endometrium, liver, oesophagus, bladder, oral cavity, thyroid, pancreas, retina and skin, preferably prostate cancer.

With a tumor sample is meant a sample taken from a tumor either prior to or after removal of the tumor from the subject bearing said tumor.

With a normal sample is meant a sample obtained for use in determining base-line expression levels. Accordingly, a normal sample may be obtained by a number of means including from non-cancerous cells or tissue e.g., from cells surrounding a tumor or cancerous cells of a subject; from subjects not having a cancer; from subjects not suspected of being at risk for a cancer; or from cells or cell lines derived from such subjects. A normal sample also includes a previously established standard, such as a previously characterized cancer cell line. Accordingly, any test or assay conducted according to the invention may be compared with the established standard and it may not be necessary to obtain a normal sample for comparison each time.

A sample can be any tissue, cell, cell extract, urine, serum, whole blood, plasma concentrate, a precipitate from any fractionation of the plasma/blood or urine such as for example exosomes (hereinafter also referred to as microvesicles), etc isolated from a subject such as for example a sample isolated from a subject having cancer or from a healthy volunteer. A "sample" may also be a cell or cell line created under experimental conditions, that is not directly isolated from a subject. A subject can be a human, rat, mouse, non-human primate, feline, etc.

For example, exosomes could be extracted from a urine, blood or serum sample from a cancer patient. Since the membranes of said exosomes are a representation of the cell membrane of the cancer cells from which they arrive, phospholipid profiling of these exosomes is a good alternative for phospholipid profiling from cancer tissue directly and more importantly a much less invasive method. This makes it much easier to follow the progression of a tumor over time, without having to take biopsies of the tumor each time. Said exosomes can for example be isolated making use of lab-on-chip technology, allowing also subsequent analysis of the phospholipid profiles, thereby allowing high-throughput screening. In the latter Exosomes (Microvesicles) can be isolated by differential centrifugation according to previous publications (Valadi H, Ekstrom K, Bossios A, Sjostrand M, Lee JJ, Lotvall JO. Exosome-mediated transfer of mRNAs and microRNAs is a novel mechanism of genetic exchange between cells. Nat Cell Biol 2007;9:654-659 - Zhang Y, Liu D, Chen X, Li J, Li L, Bian Z, Sun F, Lu J, Yin Y, Cai X, Sun Q, Wang K, Ba Y, Wang Q, Wang D, Yang J, Liu P, Xu T, Yan Q, Zhang J, Zen K, Zhang CY. Secreted monocytic miR-150 enhances targeted endothelial cell migration. Mol Cell 2010;39:133-144.). Briefly, after removing cells and other debris by centrifugation at 300g, and 16,500g, the supernatant are centrifuged at 100,000g for 70 min (all steps were performed at 4°C). Exosomes are collected from the pellet and resuspended in RNase-free water. The presence of exosomes after ultracentrifugation are determined with flow cytometry. To confirm that the vesicles are of the correct size, flow cytometry gates were set using 1 micron beads (Invitrogen), yielding microvesicles for use on microfluidic chips as described hereinbelow.

By using the *in vitro* method of the present invention, one is able to determine the lipogenicity of a tumor and subsequently predict the potential of a tumor to evolve into an aggressive tumor phenotype or to predict the potential of a tumor to respond to anti-cancer therapy. Said anti-cancer therapy including any therapy known from the art, such as for example, but not limited to chemotherapy, radiotherapy,... In addition, the *in vitro* method according to the invention is very suitable for determining the subset of patients likely to respond to therapies aiming at inhibiting the tumor lipogenesis, said therapies including but not limited to inhibitors of enzymes involved in for example fatty acid synthesis, such as FASN, acetyl-CoA carboxylase, choline kinase and ATP-citrate lyase. Said inhibitors may be used as mono-therapy or as a sensitizer used in known cancer therapies. As such, the *in vitro* method according to the invention may also be used to determine whether or not a patient responded to an anti-lipogenesis therapy.

With a tumor having an aggressive lipogenic phenotype is meant a tumor which endogenously produces fatty acids *de novo* and renders the patient with a poorer prognosis i.e. a tumor which is less responsive to anti-cancer therapy and/or having a higher potential to progress or to metastasize compared to a tumor rendering the patient with a good survival prognosis.

Phospholipids are a class of lipids that are a major component of cell membranes and that can form lipid bilayers. Most phospholipids contain a diglyceride, a phosphate group and a simple organic molecule such as for example choline in phosphatidylcholine (PC), inositol in phospatidylinostol (PI), serine in phosphatidylserine (PS) and ethanolamine in phosphatidylethanolamine (PE). A diglyceride consists of 2 fatty acid chains, covalently bound to a glycerol molecule through ester linkages. A fatty acid is a carboxylic acid having an unbranched aliphatic tail of at least 4 carbon atoms, which is either saturated or unsaturated, depending on the presence of double bonds. Saturated fatty acids are fatty acids with no double bonds in their aliphatic tail, mono-unsaturated fatty acids are fatty acids having exactly one double bond in their aliphatic tail, and poly-unsaturated fatty acids have 2, 3, 4, 5, 6, 7 or more double bonds in their aliphatic tail.

The phospholipids according to the present invention are selected from the group comprising; phosphoglycerides, phosphatidic acid, phosphatidylethanolamine, phosphatidylcholine, sphingophospholipids, phosphatidylserine and phosphoinositides. Wherein for example phosphoglycerides, comprise a phosphate group linked to the first carbon of glycerol of a diglyceride, and sphingophospholipids (e.g., sphingomyelin), wherein a phosphate group is esterified to a sphingosine amino alcohol. Another example of a sphingophospholipid is a sulfatide, which comprises an ionic sulfate group that makes the molecule amphipathic. A phopholipid may, of course, comprise further chemical groups, such as for example, an alcohol attached to the phosphate group. Examples of such alcohol groups include serine, ethanolamine, choline, glycerol and inositol. Thus, specific phosphoglycerides include a phosphatidyl serine, a phosphatidyl ethanolamine, a phosphatidyl choline, a phosphatidyl glycerol or a phosphatidyl inositol. Other phospholipids include a phosphatidic acid or a diacetyl phosphate. In one aspect, a phosphatidylcholine comprises a dioleoylphosphatidylcholine (a.k.a. cardiolipin), an egg phosphatidylcholine, a dipalmitoyl phosphalidycholine, a monomyristoyl phosphatidylcholine, a monopalmitoyl phosphatidylcholine, a monostearoyl phosphatidylcholine, a monooleoyl phosphatidylcholine, a dibutroyl phosphatidylcholine, a divaleroyl phosphatidylcholine, a dicaproyl phosphatidylcholine, a diheptanoyl phosphatidylcholine, a dicapryloyl phosphatidylcholine or a distearoyl phosphatidylcholine.

By expression level of phospholipids is meant the levels of intact phospholipids as determined by any suitable method, such as for example analyzed by ESI - MS/MS. In said methodology, the phospholipids are identified based on the intensity of the ionised species, expressed as the % intensity level of individual phospholipids, versus the total intensity level of all phospholipids measured.

With the relative expression level of phospholipids is meant the difference in expression level of phospholipids in a tumor sample compared to the expression level of phospholipids in a normal sample. In some embodiments, the relative expression level may be determined at different time points, e.g. before, during, and after therapy. The relative expression level may be expressed in any suitable way such as for example as log2.

The relative expression level of phospholipids is considered to be increased if the log2 value is higher than about 0, whereas it is considered to be decreased if the log2 value is lower than about 0. In a further embodiment the relative expression level of fatty acids is considered to be increased if the log2 value is higher than about 0,1; 0,2; 0,3; 0,4; or 0,5; and the relative expression level of fatty acids is considered to be decreased if the log2 value is lower than about -0,1; -0,2; -0,3; -0,4 or -0,5.

In an alternative embodiment of the present invention, the change in phospholipids composition is scored by determining the ratio of mono-unsaturated versus poly-unsaturated phospholipids in a sample, wherein an increase in the ratio of mono-unsaturated versus poly-unsaturated phospholipids in a tumor sample versus a normal sample is indicative for a more aggressive lipogenic phenotype of the tumor.

In a further embodiment, the *in vitro* method for determining the lipogenicity of a tumor in a subject, comprises; determining the lipogenic profile of a tumor in a patient; wherein an increase in species with one or two unsaturations (in both acyl chains together), and a decrease in PL species with more than 3 unsaturations is indicative for a more aggressive lipogenic phenotype.

In yet a further embodiment, the *in vitro* method for determining the lipogenicity of a tumor in a subject, comprises; determining the lipogenic profile of a tumor in a patient; wherein a decrease in saturated phospholipids species, an increase in species with one or two unsaturations (in both acyl chains together), and a decrease in PL species with more than 3 unsaturations is indicative for a more aggressive lipogenic phenotype.

In a further embodiment the mono-unsaturated phospholipids according to the present invention are the mono-unsaturated phosphatidylcholines (PC) with one or two mono-unsaturated fatty acyl chains, selected from the group comprising; PC28:1, PC30:1, PC30:2, PC32:1, PC32:2, PC34:1, PC34:2, PC36:1, PC36:2, PC38:1, PC38:2, PC40:1 and PC40:2., preferably PC34:1.

In a further embodiment, the poly-unsaturated phospholipids are poly-unsaturated phosphatidylcholines (PC), selected from the group comprising; PC32:3, PC34:2, PC34:3, PC34:4, PC36:3, PC36:4, PC36:5, PC36:6, PC38:2, PC38:3, PC38:4, PC38:5, PC38:6, PC38:7, PC40:2, PC40:3, PC40:4, PC40:5, PC40:6, PC40:7, PC40:8, PC42:2, PC42:3, PC42:4, PC42:5, PC42:6, PC42:7, PC42:8, PC42:9, PC42:10, PC42:11, PC44:2, PC44:3, PC44:4, PC44:5, PC44:6, PC44:7, PC44:8, PC44:9, PC44:10, PC44:11, and PC44:12; preferably PC36:3, PC38:3, PC36:4, PC38:4, PC40:4, PC36:5, PC38:5, PC40:5; and most preferably PC36:4 and/or PC38:4.

In a preferred embodiment, the mono-unsaturated phosholipids are the mono-unsaturated phosphatidylcholines (PC) PC34:1; and the poly-unsaturated phospholipids are the poly-unsaturated phosphatidylcholines (PC) PC36:4 and/or PC38:4.

In a further embodiment, the mono-unsaturated phospholipids are the phosphatidylethanolamines (PE) with one or two mono-unsaturated fatty acyl chains, selected from the group comprising; PE32:1 and PE34:2.

In yet a further embodiment, the poly-unsaturated phospholipids are poly-unsaturated phosphatidylethanolamines (PE), selected from the group comprising; PE36:4, PE38:4 and PE40:4.

In a further embodiment the mono-unsaturated phospholipids are the phosphatidylserines (PS) with one or two mono-unsaturated fatty acyl chains, selected from the group comprising; PS36:2, PS38:2, PS40:2, and PS42:2.

In yet a further embodiment the poly-unsaturated phospholipids are poly-unsaturated phosphatidylserines (PS), selected from the group comprising; PS38:4, PS40:4, PS38:5, PS40:5 and PS38:6.

In a further embodiment the mono-unsaturated phospholipids are the phosphatidylinositides (PI) with one or two mono-unsaturated fatty acyl chains, selected from the group comprising; PI34:1, PI36:1, PI38:1, PI34:2, PI36:2 and PI38:2.

In yet a further embodiment the poly-unsaturated phospholipids are poly-unsaturated phosphatidylinositides (PI), selected from the group comprising; PI36:4 and PI38:4.

In a further embodiment, in addition to determining the expression of phospholipids, the relative expression level of other biomarkers for an aggressive lipogenic phenotype may be determined, such as for example, but not limited to, FASN (fatty acid synthase), ACCA (acetyl CoA carboxylase alpha), choline kinase and ACLY (ATP citrate lyase) expression or phosphorylation/activation. A tumor having an aggressive lipogenic phenotype may than for example be identified by an increase in relative expression level of mono-unsaturated phospholipids, a decrease in relative expression level of poly-unsaturated phospholipids, and an increase in expression of one or more other biomarker for an aggressive lipogenic phenotype. Alternatively, a tumor having an aggressive lipogenic phenotype may for example be identified by an increased ratio of mono-unsaturated versus poly-unsaturated phospholipids in the tumor sample compared to the normal sample, and an increase in expression of one or more other biomarker for an aggressive lipogenic phenotype.

The invention further relates to the use of the *in vitro* method according to the present invention for determining the lipogenicity of a tumor in a subject.

Finally, the invention provides a kit for performing the *in vitro* method according to this invention, said kit comprising the reagents for the ESI - MS/MS or other mass spectrometry-based sample preparation of phospholipids, in particular an antioxidant, solvents and standards.

In a particular embodiment said kit comprises a microfluidic chip, and a coated surface for the immobilization of microvesicles to a surface coated with molecules or agents having an affinity for said microvesicles or being capable of binding microvesicular particles.

Any molecule which has an affinity for microvesicles suitable for coating the selected surface material can be used. With a molecule having an affinity for microvesicles is meant that such molecule is capable of binding covalently or non-covalently to a molecule present on a microvesicle. Preferably said molecule present on a microvesicle is a membrane-bound molecule. Preferably, molecules having a high affinity for a microvesicle are used. Preferably, affinity is expressed as a dissociation constant. Preferably, molecules having a dissociation constant lower than 0.1 nM for microvesicles are used, more preferably lower than 10 nM. More preferably, molecules having a dissociation constant lower than 10⁻¹⁵ M for microvesicles are used. In another preferred embodiment, an affinity for a microvesicle is used with a dissociation constant in a range between 0.1-10 nM. Methods of determining affinity are known in the art. Preferably, a method is used as described in Johnson et al. Journal of Molecular Biology 368 (2): 434-449.

Any surface that is suitable for immobilization using coatings having an affinity for microvesicles can be used Preferred surfaces are made of material comprising glass, mica, plastic, metal or ceramic materials. There are various methods known for coating surfaces having affinity for (glyco)-proteins, cell membranes or biomolecules in general. Typically, these methods use a reactive group which binds covalently or non-covalently to a certain biomolecule. For example, slides coated with aminoproplylsilane are used for non-covalent adsorption of protein. Epoxysilane coated slides are reactive with lysine, arginine, cysteine and hydroxyls at pH 5-9. Aldehyde coated slides are reactive with lysine and arginine where pH 7-10 drives Schiff's base reaction. A skilled person will know how to select the right coating suitable for use in combination with the selected surface material and test the affinity for microvesicles.

The resulting coated surface is put into contact with microvesicles by applying a laminar flow to a fluid comprising said microvesicles. Said fluid can be any fluid that is compatible with microvesicles. With compatible is meant that the integrity of the microvesicles remains intact, which means that at least phospholipids used in the methods of the present invention are present within the microvesicles. Preferably said fluid comprises plasma, cell culture medium, phosphate buffered saline (PBS), phosphate buffered potassium, or 4-(2-hydroxyethyl)-1-piperazineethanesulfonic acid (HEPES).

A laminar flow is a flow regime characterized by high momentum diffusion, low momentum convection, pressure and velocity independent from time. Said laminar flow is characterized by a Reynolds number of less than 2000 and higher than 0. Preferably, laminar flows with a Reynolds value between 0 and 1000, more preferably between 0 and 500 and most preferably between 0 and 100. A skilled person will know how to achieve a laminar flow. Any method capable of applying a fluid in a laminar flow to said coated surface can be used. Preferred is a laminar flow which is linear in one direction. Preferred is a laminar flow that optimizes the contact area, contact time and flow speed to the functionality of the fluidic device. Further preferred is a laminar flow through a channel comprising a functionalized wall.

Preferably, a method is used wherein the affinity for the microvesicles is specific. An advantage of a specific affinity is that binding of undesired molecules or particles is reduced. Preferred methods are methods in which an affinity linker is bound covalently or non-covalently to the surface. An affinity linker is a molecule that is capable to covalently or non-covalently bind to a binding partner, resulting in a complex between said affinity linker and said binding partner. Said binding partner can be a molecule capable of binding to a microvesicle or it can be a molecule present on the microvesicle that can directly interact with said affinity linker. Examples of affinity linkers and their binding partners are;Streptavidin or avidin and biotin; an antibody and antigen; a ligand and receptor, lectin and saccharide, protein A and/or protein G-immunoglobulin constant region, and Tag peptide sequence and Tag antibody. The terms affinity linker and binding partner refer to their function. Therefore, depending on how the above mentioned affinity linker-binding partner combinations are used, the terms are exchangeable. For example a biotin can be an affinity linker if it is bound to the surface or it can be a binding partner when it is bound to the microvesicle. Any method to bind an affinity linker to a surface can be used. Methods to bind an affinity linker to a coated surface differ depending on the material of surface and the nature of the affinity linker. A skilled person will be able to select the correct method suitable for the type of surface material and affinity linker of choice. Methods to bind an affinity linker to a surface are known to a skilled person. Methods to bind antibodies to metal or silicon surface are well known in the art. Preferred methods are described in Bioelectrochemistry Volume 66, Issues 1-2, April 2005, Pages 111-115 . Methods to bind antibodies to glass surface are also known and described in J Colloid Interface Sci. 2002 Aug 1;252(1):50-6. In a particular embodiment the affinity linkers are selected from the group consisting of antibody species, proteins, aptamers, surfaces selectively restricting microvesicles from passage, and surfaces with selective adhesion to microvesicles; wherein the proteins are particularly selected from the list comprising lectin, or other sugar binding compounds; and wherein the lectin is particularly selected from the group comprising GNA, NPA, Concanavalin A, or cyanovirin.

In a more particular embodiment, said laminar flow is applied using microfluidics. The term microfluidics refers to devices, systems, and methods for the manipulation of fluid flows with characteristic length scales in the micrometer range up to 1 millimeter (see for a more complete overview: Manz, A. and Becker. H. (Eds.), Microsystem Technology in Chemistry and Life Sciences, Springer-Verlag Berlin Heidelberg New York, ISBN 3-540-65555-7). An advantage is that microfluidic systems possess the capability to execute operations more quickly than conventional, macroscopic systems, while consuming much smaller amounts of chemicals and fluids.

In a particular embodiment of the present invention said laminar flow is created using a microfluidic chip. Preferably said microfluidic chip comprises at least one microfluidic channel with an inlet and an outlet, wherein said at least one microfluidic channel has at least one gap at a surface of said microfluidic chip enabling contact between said at least one channel and a surface. An advantage of said microfluidic chip is that it enables direct contact between a fluid in said microfluidic channel and a surface. Preferably said inlet and/or said outlet are positioned at a different surface of said microfluidic chip than the surface comprising said gap in said microfluidic channel. An advantage thereof is that this enables clamping between said microfluidic chip and said surface, while maintaining access to said inlet and/or outlet. In a preferred embodiment, holes are provided in said microfluidic chip, enabling screws or other means to attach a surface to said microfluidic chip. An advantage thereof is that a surface can be attached to said microfluidic chip to achieve a contact which prevents leakage of a fluid from said microfluidic channel. An advantage of using a microfluidic channel is that the geometry of the channel enables a controllable inducement of a laminar flow. Preferably, said channel has a height which is less than 1 mm. An advantage thereof is that the surface to volume ratio of said fluid over said coated surface is optimized. In another preferred embodiment, the channel height above the coated area is less than the channel height in other parts of the microfluidic device. An advantage thereof is that the surface to volume ratio of said fluid over the coated area is optimized, while maintaining a flow through. More preferably, said channel height is less than 0.5 mm. An advantage thereof is that this height is optimal for fluids having a viscosity of plasma. Preferably, said channel has a width of less than 1 mm. An advantage thereof is that this results in a minimal contact area of said coated surface which is in contact said fluid. Preferably, said minimal contact area is smaller than said coated surface area. Preferably, said minimal contact area is between 100 and 10000 square micrometer. In a preferred embodiment, said minimal contact area is less than 1 square millimeter. An advantage thereof is that this limits the surface area inspection time and increases the concentration of collected vesicles per surface area. More preferably, said minimal contact area is between 1 square micrometer and 0.1 square millimeter.

As is known to the skilled artisan, the aforementioned channels may further comprise a filter which allows particles smaller than a microvesicle to pass through. In this method, microvesicles are collected on the surface of said filter, a change of flow direction is then applied to re-suspend said microvesicles. This method further comprises a step of resuspending said microvesicles in a fluid before allowing said fluid comprising said microvesicles to contact said coated surface. Accordingly, in an even further embodiment the microfluidic chip as used herein comprises a pumping system. Any system suitable of pumping fluid inside a microfluidic circuit can be used. Examples are described in PHYSICS AND APPLICATIONS OF MICROFLUIDICS IN BIOLOGY David J. Beebe, Glennys A. Mensing, Glenn M. Walker Annual Review of Biomedical Engineering, August 2002, Vol. 4, Pages 261-286 .

When using microfluidic chips in the methods of the present invention, detection of microvesicles may be done using imaging techniques. An advantage of this is that this allows measurements of one or more parameters comprising but not limited to particle geometry, shape, roughness, light scattering or dimensions of microvesicles or the presence of molecules on the surface of or inside microvesicles can be determined. Any method of imaging can be used in the method. Preferred methods of imaging comprise fluorescence microscopy, including internal reflection fluorescence microscopy, electron microscopy (EM), confocal microscopy, light scattering or surface plasmon microscopy, Raman spectroscopy, ellipsometry/reflectometry, infrared spectroscopy or atomic force microscopy (AFM), or combinations thereof.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### EXAMPLES

The following examples illustrate the invention. Other embodiments will occur to the person skilled in the art in light of these examples.

### Example 1: De novo lipogenesis in prostate cancer cells is associated with increased levels of mono-unsaturated fatty acids and decreased levels of polyunsaturated fatty acids in cancer cells.

### MATERIALS AND METHODS

### Cell Culture and Treatments

LNCaP and COS-7 cells were obtained from the American Type Culture Collection (Manassas, VA). Cells were cultured at 37°C in a humidified incubator with a 5 % CO₂/95 % air atmosphere in RPMI 1640 medium, supplemented with 10 % FCS (Invitrogen, Carlsbad, CA). Cell lines were tested for authentication by checking morphology and karyotyping. Soraphen A (soraphen), which was purified from the mycobacterium *Sorangium cellulosum,* was kindly provided by Drs. Klaus Gerth and Rolf Jansen (Helmholtz-Zentrum fur Infektionsforschung, Braunschweig, Germany) (Bedorf et al., 1993; Gerth et al., 1994).

### 2-¹⁴C-acetate Incorporation Assay and TLC Analysis

LNCaP cells were treated with soraphen or vehicle (ethanol) for 24 hours. The last 4 hours, PC-labeled acetate (57 mCi/mmol; 2 µCi/dish; Amersham International, Aylesbury, UK) was added to the culture medium. Lipids were extracted according to a modified Bligh-Dyer method as previously described (De Schrijver et al., 2003). To analyze the incorporation of 2-¹⁴C-acetate into different lipid classes, TLC analysis was performed and the lipids were exposed to a PhosphorImager screen for quantification (Molecular Dynamics, Sunnyvale, CA), as previously described (De Schrijver et al., 2003).

### Clinical Tissue Specimens

Fresh, snap-frozen prostate cancer tissues and matching normal samples were obtained from patients who had undergone a radical retropubic prostatectomy for localized prostatic carcinoma. The normal and tumor tissues were identified by histological analysis of areas adjacent to the tissue that was used for lipid and western blotting analysis.

### Quantification of Total Cellular Phospholipids, Triglycerides and Cholesterol

Quantification of phospholipids, triglycerides and cholesterol was performed on Bligh-Dyer lipid extracts (De Schrijver et al., 2003) using previously described methods (Van Veldhoven and Bell, 1988; Van Veldhoven et al., 1998; Van Veldhoven et al., 1997), with minor modifications.

### Analysis of Intact Phospholipid Species by ESI-MS/MS

To prepare lipid extracts for ESI-MS/MS analysis, the tissue or cells were homogenized in 1.6 ml of 0.1 N HCl:CH₃OH 1:1 (v/v). CHCl₃ (0.8 ml) and 200 µg/ml of the anti-oxidant 2,6-di-tert-butyl-4-methylphenol (Sigma) were added (Milne et al., 2006). After addition of the lipid standards, the organic fractions were collected by centrifugation at 200 g for 5 min. Samples were evaporated and reconstituted in CH₃OH:CHCl₃:NH₄OH (90:10:1.25, v/v/v) and the lipids were analyzed by electrospray ionization tandem mass spectrometry (ESI-MS/MS) on a hybrid quadrupole linear ion trap mass spectrometer (4000 QTRAP system; Applied Biosystems, Foster City, CA) equipped with a robotic nanoflow/ion source (Advion Biosciences). The system was operated in the MRM mode for quantification of individual species. Data were expressed as fold change relative to the control samples (untreated cells or matched normal tissue) and were presented as heatmaps using the Heatmap Builder software (Clifton Watt, Stanford University, USA).

### RESULTS

To study the impact of tumor-associated *de novo* lipogenesis on cellular lipid composition, we treated LNCaP prostate cancer cells, which have high lipogenic activity, with soraphen A, a known inhibitor of fatty acid synthesis (Beckers et al., 2007). Inhibition of fatty acid synthesis, and as such *de novo* lipogenesis was confirmed by 2-¹⁴C-acetate incorporation (figure 1A).

Subsequently, total cellular lipid extracts were subjected to electrospray ionization tandem mass spectrometry (ESI-MS/MS) in order to analyze all of the major intact phospholipids species. As shown in figure 1B, there were dramatic changes in the individual phosphatidylcholine (PC) species. Soraphen treatment substantially decreased the PC species with one degree of unsaturation up to four-fold. Since PC species with two unsaturations can represent molecules with two mono-unsaturated chains, as well as molecules with one saturated and one double-unsaturated chain, soraphen treatment had an ambiguous or minor net effect on these species. Overall, the level of PC species with more unsaturations (>three) was increased up to eight-fold, depending on the species. The shift towards polyunsaturation could also be detected when lipogenesis was inhibited in 22Rv1 cells, another lipogenic prostate cancer cell line (data not shown).

To assess whether the lipogenic phenotype of cancer cells is also associated with an increased saturation of phospholipids *in vivo*, prostate tumor specimens and normal matching control tissues were analyzed for overexpression of fatty acid synthase (FASN) by western blot analysis, and the phospholipid profiles were recorded. Three out of the five matched samples (tumor versus control) showed a substantial overexpression of FASN in tumor tissue (Figure 2A). Analysis of the phospholipid composition by ESI-MS/MS demonstrated a dramatically different profile for PC in the lipogenic tumors, as compared to the non-lipogenic tumors (figure 2B). Tumors having low levels of FASN expression (patients 4 and 5) had an overall increase in polyunsaturated species and a reduction in mono-unsaturated species, as compared to matching normal tissue. However, the tumors with increased FASN expression (patients 1, 2 and 3) showed the reverse: there was a consistent increase in mono-unsaturated acyl chains and a decrease in polyunsaturated species in tumor tissue, as compared to matching normal tissue (Figure 2B). These data support our findings with the lipogenesis inhibitor soraphen and provide *in vivo* evidence that tumor-associated lipogenesis increases the saturation of phospholipids in human tumors.

### Conclusion

In conclusion these data indicate that *de novo* lipogenesis in cancer cells provides cells with saturated and mono-unsaturated acyl chains, which replenishes the cells with membrane components and simultaneously increases the relative degree of saturation of phospholipids, particularly that of Phosphatidylcholine (PC).

### Example 2: Modulation of de novo lipogenesis in prostate cancer cells affects multiple parameters associated to a more aggressive lipogenic phenotype

### MATERIALS AND METHODS

### Cell Culture and Treatments

See example 1

### Analysis of Intact Phospholipid Species by ESI-MS/MS

See example 1. For the palmitic acid rescue experiments, palmitic acid (Sigma, St. Louis, MO) was complexed to fatty acid-free bovine serum albumin (BSA) (Invitrogen, Carlsbad, CA), as previously described (Brusselmans et al., 2005).

### Lipid Peroxidation Product Assay

Equal amounts of cells were scraped in ice-cold PBS, kept on ice for 15 min, and then sonicated (10 bursts). After centrifugation at 3,000 g for 10 min, the supernatants were analyzed using a lipid peroxidation assay kit (Oxford Biomedical Research, Oxford, MI) that quantifies the amount of malondialdehydes and 4-hydroxyalkenals, which are generated from fatty acid peroxide decomposition.

### CD36-binding Assay

LNCaP cells were cultured in the presence or absence of soraphen and/or palmitic acid for 72 hr. The cells were exposed to H₂O₂ (300µM) for 30 min. Cells were trypsinized and labeled with Cell Tracker Dye Orange CMRA (C34551). The labeled LNCaP cells (1.5 x 10⁵) were subsequently layered over a monolayer of COS-7 cells, which were cultured on glass coverslips and transfected with a CD36-encoding construct (pCD36) or the corresponding empty vector (pEF-BOS) (both kindly provided by R. Thorne, University of Newcastle, Australia). Cells were co-cultured in 1 ml of RPMI medium for 1 hr at 37°C. After extensive washing, the cultures were fixed and imaged. The fluorescence signal of the CellTracker Dye was measured using Adobe Photoshop software (San Jose, CA).

### Determination of Flip-flop Rate and Doxorubicin Accumulation

To measure the flip-flop rate of doxorubicin, cells were treated with soraphen or vehicle for 72 hr. During the last 24 hr of treatment, 10 µM of NBD-phosphatidylethanolamine (NBD-PE; Invitrogen) was added. Verapamil was added at a concentration of 100 µM 1 hr prior to harvesting the cells by trypsinization. The doxorubicin flip-flop rate across the plasma membrane was measured as the reduction in fluorescence of NBD due to quenching of NBD-PE by doxorubicin (Regev et al., 2005) (See Supplemental Methods for details).

To visualize the intracellular doxorubicin accumulation, doxorubicin was added at a final concentration of 10 µM. After 30 min, the cells were analyzed using a fluorescence microscope (515 nm longpass emission filter) and the LIDA software (Leica Microsystems GmbH).

To measure doxorubicin accumulation fluorimetrically, cells were exposed to 10 µM of doxorubicin for 30 min, washed in PBS, lysed in 0.3 M HCl in 50 % ethanol (v:v), and centrifuged at 700 g for 5 min. The fluorescence of doxorubicin was measured at an excitation wavelength of 490 nm and an emission wavelength of 580 nm. Where indicated, media were supplemented with palmitic acid.

### Cell Death Assay

At the indicated time after exposure to soraphen and/or other compounds, the adherent and floating cells were collected by trypsinization and centrifugation and both cell populations were combined. The viable and dead cells were counted using the trypan blue dye exclusion assay, as previously described (De Schrijver et al., 2003).

### Statistical Analysis

The results were analyzed by one-way ANOVA using a Tukey post hoc test. P-values <0.05 were considered to be statistically significant. All data presented represent means ± SE, as indicated in the figure legends.

### RESULTS

### Modulation of De novo Fatty Acid Synthesis in Cancer Cells Affects the Susceptibility of Cellular Membranes to Lipid Peroxidation

Saturated and polyunsaturated acyl chains dramatically differ in terms of their structural and physiochemical properties. One of the key differences is their susceptibility to peroxidation. In this process, free radicals extract electrons from lipids in cellular membranes, which leads to the formation of oxidized lipid species. These lipid species have important biological functions and may ultimately degrade into smaller reactive products including malondialdehydes (MDA) and 4-hydroxyalkenals, which can cause cell damage when expressed at high levels (Deininger and Hermetter, 2008; Rabinovich and Ripatti, 1991; Schneider et al., 2008). As the hydrogens in between double bonds in methylene (CH2) groups are particularly reactive, polyunsaturated acyl chains are much more susceptible to peroxidation. Based on our finding that modulation of *de novo* fatty acid synthesis in cancer cells affects the balance between saturated and polyunsaturated acyl chains in the phospholipids of cellular membranes, we assessed whether modulation of this metabolic pathway affected the susceptibility of cancer cells to radical-induced lipid peroxidation. LNCaP cells were treated with soraphen for 3 days and then exposed to H₂O₂. The products of lipid peroxidation were colorimetrically measured. Soraphen treatment significantly increased the levels of lipid peroxidation products (Figure 3), which was consistent with the shift towards phospholipid polyunsaturation observed with soraphen treatment. Treatment with exogenous H₂O₂, which produces higher levels of free radicals, induced a further increase in peroxidation products, indicating that soraphen mediated a sensitization effect. Interestingly, partial replenishment of saturated acyl chains by supplementation of the medium with exogenous palmitic acid largely reversed these changes, supporting the idea that enhanced lipogenesis renders cancer cells less susceptible to lipid peroxidation by limiting the degree of phospholipid polyunsaturation.

### De novo Fatty Acid Synthesis Affects CD36-mediated Cell Recognition of Cancer Cells

Oxidized phospholipids in cellular membranes are known to undergo a conformational change that forces oxidized acyl chains to protrude from the interior of the hydrophobic membrane into the polar aqueous environment where they can function as endogenous pattern recognition ligands (Hazen, 2008). Exposed cell surface oxidized phospholipids, particularly the oxidized PC species, may be recognized by the scavenger receptor CD36. This receptor is expressed for instance on innate immune cells that are involved in the surveillance of host tissues and in the clearance of damaged, senescent or apoptotic cells. Since we determined that inhibition of *de novo* lipogenesis in cancer cells increased the degree of polyunsaturation of phospholipids and their susceptibility to peroxidation, we examined whether modulation of lipogenesis affected the recognition of cancer cells by CD36-positive cells. LNCaP cells either cultured in the presence or absence of soraphen, were overlaid onto COS cells that had been transfected with a CD36-expression construct (pCD36) or an empty vector (pEF-BOS) (Thorne et al., 1997). After extensive washing, the bound LNCaP cells were counted. As demonstrated in Figure 4A, soraphen treatment followed by a brief exposure to H₂O₂ substantially increased the number of CD36-bound LNCaP cells. Addition of exogenous palmitic acid counteracted these effects. These findings suggest that tumor-associated lipogenesis limits the exposure of oxidized polyunsaturated species on the cell surface by enhancing the degree of saturation of membrane phospholipids, particularly that of PC. This response may minimize the recognition of tumor cells by CD36-positive cells and may potentially facilitate the evasion of lipogenic cancer cells from the immune system (Hazen, 2008).

### De novo Lipogenesis Determines the Sensitivity of Cancer Cells to Oxidative Stress-induced Cell Death

There is growing evidence that oxidized phospholipids and their degradation products play a key role in the induction of cellular apoptosis (Dupertuis et al., 2007; Fruhwirth and Hermetter, 2008; Tang et al., 2002; West et al., 2004). We therefore investigated whether modulation of *de novo* lipogenesis affected the sensitivity of cancer cells to oxidative stress-induced cell death. The native LNCaP cells were fairly resistant to H₂O₂-induced cell death, but pretreatment with soraphen markedly increased their death in response to H₂O₂, as demonstrated by trypan blue staining (Figure 4B). These effects were counteracted by exogenous palmitic acid. Interestingly, when the medium was supplemented with a mixture of saturated and polyunsaturated fatty acids, the rescue effect was much less pronounced. Overall, these data support the idea that increased lipogenesis protects cancer cells from oxidative stress-induced cell death by changing the extent of saturation of cellular membranes.

### Tumor-associated Fatty Acid Synthesis Affects the Uptake and Response to Common Chemotherapeutics

In addition to its effect on lipid peroxidation, modulation of membrane lipid composition is known to have a major impact on the mobility of membrane components (Rabinovich and Ripatti, 1991; Stillwell and Wassall, 2003). Transversal mobility of membrane components, also referred to as flip-flop, occurs at a low rate unless it is facilitated by specific transporters. However, for certain exogenous compounds including commonly used chemotherapeutics, such as doxorubicin, passive flip-flop is a major mechanism of entry into the cells (Regev et al., 2005). Since treatment of cells with exogenous polyunsaturated fatty acids is known to promote the uptake of doxorubicin (Davies et al., 1999), we determined whether inhibition of fatty acid synthesis would promote membrane flip-flop and doxorubicin uptake. To measure the flip-flop of doxorubicin, soraphen or vehicle-exposed LNCaP cells were treated with 7-nitrobenzo-2-oxa-1,3-diazole phosphatidylethanolamine (NBD-PE), a fluorescently labeled phospholipid that incorporates into both leaflets of the plasma membrane. After addition of doxorubicin, there was a rapid quenching of NBD fluorescence, which is caused by the association of doxorubicin with phospholipids in the outer leaflet. As doxorubicin translocates to the inner leaflet, further quenching of NBD is observed (Regev et al., 2005). Monitoring of NBD quenching indicated that soraphen induced a six-fold increase in the flip-flop rate of doxorubicin (Figure 5A). These effects were not caused by indirect effects of P-glycoprotein which pumps hydrophobic molecules such as doxorubicin out of the cell, since similar results were obtained in the presence of the P-glycoprotein inhibitor verapamil (Figure 5A). The increased flip-flop rate was accompanied by a significant increase in the intracellular accumulation of doxorubicin, as assessed by fluorescence microscopy and fluorimetric analysis of cellular extracts (Figure 5B). Addition of exogenous palmitic acid counteracted the effects of soraphen (Figure 5B). Soraphen treatment markedly sensitized LNCaP cells to the cytotoxic effects of doxorubicin, which was consistent with the increased accumulation of doxorubicin in soraphen-treated cells and their increased susceptibility to cell death. LNCaP cells grown under standard culture conditions were fairly resistant to doxorubicin-induced cell death (8 % cell death at 4 µM) (Figure 5C), but pretreatment with soraphen, which alone induced death in up to 20 % of the cells, increased the rate of cell death to approximately 50%. This potent effect was synergistic, as assessed using the Combination Index (CI) method (Chou and Talalay, 1984). For all tested ratios of doxorubicin/soraphen (80:1; 40:1; 20:1 and 10:1), the CI values were less than 1. The combination used in the panel C experiment (4 µM doxorubicin, 100 nM soraphen) was strongly synergistic (0.1 < CI < 0.3) (data not shown). Addition of exogenous palmitic acid counteracted these effects and largely rescued the cells from death (Figure 5C). Similar results were obtained with the prostate cancer cell line 22Rv1 (data not shown). Collectively, these data indicate that *de novo* lipogenesis in cancer cells affects the uptake of chemotherapeutics and modulates the response of cancer cells to these agents.

### DISCUSSION

In summary, the findings from examples 1 and 2 demonstrate that cancer cells become more autonomous in terms of their lipid supply and simultaneously shift their lipid composition towards increased saturation by activating *de novo* lipogenesis. Furthermore, said tumor-associated lipogenesis confers a significant advantage to cancer cells, as it helps them to survive both carcinogenic- and therapeutic-mediated insults. Therefore, phospholipid profiling makes it possible to identify lipogenic, more aggressive and resistant tumors, based on the determination of phospholipid saturation.

### Example 3: Phospholipid profiling in an extended group of patients making use of PC, PE, PS and PI profiles

### Tissue collection

Prostate tumor tissues and matching normal samples were obtained from 14 patients who had undergone a radical retropubic prostatectomy for localized prostatic carcinoma Prostate tissue specimens were taken using a 6 or 8mm diameter punch biopsy instrument. Samples were snap-frozen in liquid nitrogen and stored at -80°C for lipid, protein and RNA extractions. Normal and tumor tissues were identified by histological analysis of areas adjacent to the tissue that were embedded in Tissue-Tek OCT (Miles Inc, Westhaven, CT.) Serial sections were processed for hematoxylin and eosin staining. The cancer samples were evaluated for their Gleason scores and the percentage of cancer they contain was estimated.

### Determination of DNA concentration

Determination of the DNA concentration is used to normalize the amount of standards and running solution added to the samples used for lipid analysis. Samples were sonicated and diluted in homogenization buffer (5x10-2M Na2HPO4/NaH2PO4 buffer pH 7,4; 2M NaCl and 2x 10-3 M EDTA). Herring sperm DNA (Promega, Madison, WI; 0-5µg DNA/125µl) was used to create a standard curve by making different dilutions in homogenization buffer. Next, samples were incubated for one hour at 37°C to improve lysis. Thereafter, 2µg/ml Hoechst 33258 reagent (Calbiochem, La Jolla, CA) was added.

DNA content of the samples and the herring sperm DNA were measured using a fluorimeter (Fluostar SLT, BMG Labtech, Offenburg, Germany). Excitation: 355ηm; emission: 460ηm. The DNA content of each sample was calculated based on the data of the standard curve.

### Lipid extraction

Lipid extracts of the samples were made by homogenizing approximately 40mg of tissue in 800µl PBS with a Dounce homogenizer. An aliquot of 100µl was set aside for DNA analysis. The remaining 700µl was transferred to a glass tube with Teflon liner and 900µl 1N HCl:CH₃OH 1:8 (v/v), 800µl CHCl₃ and 500µg of the antioxidant 2,6-di-tert-butyl-4-methylphenol (BHT) (Sigma, St. Louis, MO) were added. The appropriate lipid standards were added based on the amount of DNA of the original sample (per mg DNA: 150ηmol PC 26:0; 50ηmol PC 28:0; 150ηmol PC 40:0; 75ηmol PE 28:0; 8,61ηmol PI 25:0 and 3ηmol PS 28:0). After mixing for 5min in a rotary shaker and phase separation (high speed centrifugation at 17300g, for 5min at 4°C), the lower organic fraction was collected using a glass Pasteur pipette and evaporated using a Savant Speedvac *spd111v* (Thermo Fisher Scientific, Waltham, MA). The remaining lipid pellet was stored at-20°C.

### Mass spectrometry

For mass spectrometry (MS), lipid pellets were reconstituted in running solution (CH₃OH:CHCl₃:NH₄OH; 90:10:1.25, v/v/v) depending on the amount of DNA of the original tissue sample (1µl running solution/1µg DNA). PL species were analyzed by electrospray ionization tandem mass spectrometry (ESI-MS/MS) on a hybrid quadrupole linear ion trap mass spectrometer (4000 QTRAP system; Applied Biosystems, Foster City, CA) equipped with an Advion TriVersa robotic nanoflow/ion source device for automated sample injection.(Advion Biosciences, Ithaca, NY). Before measurement, samples were diluted in running solution. A dilution of 1/30 was used for measurement of the phosphatidylcholine (PC), phosphatidylserine (PS) and phosphatidylinositol (PI) species. For analysis of phosphatidylethanolamine (PE) species a 1/3 dilution was used.

PL profiles were recorded in positive and negative ion scan mode at a collision energy of 50eV for precursor (prec.) 184, 35eV for neutral loss (nl.) 141, -40eV for nl. 87 and - 55eV for prec. 241 for PC, PE, PI and PS species respectively. For quantification of individual species the system was operated in the MRM mode. Typically, a 3min period of signal averaging was used for each spectrum. Data were corrected for carbon isotope effects and are expressed as the percentage of total measurable PL species of the same PL family. Only the PL species which account for more than 0,1% of the total amount of measured phospholipids of the same family are shown in the graphs.

### Cluster analysis

Clustering analysis was carried out by using a centroid linkage clustering algorithm of the Cluster 3.0 software [Eisen, M.B., P.T. Spellman, P.O. Brown, and D. Botstein, Cluster analysis and display of genome-wide expression patterns. Proc Natl Acad Sci U S A, 1998. 95(25): p. 14863-8.]. The clustering results were visualized using the Java TreeView 1.1.5. software *[*Saldanha, A.J., Java Treeviewextensible visualization of microarray data.Bioin formatics, 2004. 20(17): p. 3246-8*.].*

### RESULTS

### Characteristics of tumor specimens

To study changes in PL profiles in cancer tissue versus normal tissue and among tumor tissues of individual patients, approximately 40mg of prostate tumor tissue and adjacent normal prostate tissue from 14 prostate cancer patients, who had undergone a radical retropubic prostatectomy for localized prostatic carcinoma, was obtained. All tumor tissues were verified by histological examination to consist for at least 80 percent of tumor tissue.

### Optimization of the phospholipid profiling procedure

To look at intact PL species we used a mass spectrometry-based approach that was developed in house in collaboration with Prof. R. Derua and Prof. E. Waelkens (Department of Molecular Cell Biology, K.U.leuven). This procedure was based on a protocol described by Brügger et al [Brugger, B. , G. Erben, R. Sandhoff, F. T. Wieland, and W. D. Lehmann,Quantitative analysis of biological membrane lipids at the low picomole level by nano-electrospray ionization tandem mass spectrometry. Proc Natl Acad Sci U S A, 1997. 94(6): p. 2339-44*.]* and Milne et al *[*Milne, S., P. Ivanova, J. Forrester, and H. Alex Brown, Lipidomics: an analysis of cellular lipids by ESI-MS. Methods, 2006. 39 (2) : p. 92-103*.*] and was adapted for use on an ABI 4000 QTRAP mass spectrometer equipped with a Triversa robotic nanoflow/ion source device for automated sample injection.

As this protocol was developed for PL analysis of cultured cells, we adapted it for use on clinical samples. Briefly, the homogenization procedure was optimized by varying the amount of starting material and by modifying the procedure (number of strokes) for Dounce homogenization. The MS procedure was optimized by varying the dilution of the lipid extracts.

### Phospholipid profiling of prostate cancer tissues and matching normal tissues

In order to detect individual classes of phospholipids directly from total lipid extracts in a shotgun approach, different precursor ion and neutral loss scans were run in a tandem mass spectrometric (MS/MS) analysis. This approach enables to focus on specific classes of lipids, reducing the complexity of the spectra, and eliminates baseline noise. Four major PL classes were analyzed: PC, PE, PS and PI. PC species were detected in positive ion mode in a precursor scan for m/z 184, corresponding to the phosphocholine head group peak after fragmentation in MS/MS mode. PE species were detected by collision-induced decomposition in positive ion mode yielding an ion corresponding to the nl. of phosphoethanolamine. PS and PI were measured in negative ion mode scan for nl.87 and prec. 241, respectively.

Quantification of species was done in multiple reaction monitoring (MRM) mode, after isotope correction, focusing on the most abundant PL species.

For optimal identification of the degree of unsaturation, amounts of lipid species were not expressed in absolute values but rather as the percentage of all measurable species within one class of phospholipids (e.g. PC). To avoid errors due to differences in ionization efficiency, differences in the percentages were indicated as a ratio of values in cancer tissue compared to matching normal tissue. A log2 scale was used to equalize differences in both directions (increase and decrease). To avoid errors due to background noise species accounting for less than 0,1% of the total intensity of all species of one PL family were not taken into account.

Relative changes in PL species in prostate tumor versus normal prostate tissue from the 14 prostate cancer patients were determined. Marked differences were observed in PL profiles in 13 out of 14 patients. Interestingly, different patterns of changes were observed among different patients (data not shown). Some changes (e.g. an increase in PS 40:8) were observed in all patients. Most other changes were restricted to specific subsets of patients (e.g. increase in PE 38:0). To better reveal differences and similarities in PL profiles among the different patients, cluster analysis was performed. This revealed recurrent changes and divided the patients in 2 major groups. Figure 6 represents the average increase/decrease (log2) for each of the tested phospholipids species.
Cluster A (Fig. 6A) (patients 3, 4, 5, 6, 7, 9, 10, 11 and 13) is characterized by a marked decrease in fully saturated PL species, an increase in species with one or two unsaturations (in both acyl chains together), and a decrease in PL species with more than 3 unsaturations. This pattern is most outspoken in the PC fraction and will be referred to as the 'lipogenic profile' as it can be largely explained by the lipogenic switch (*vide infra*).
Cluster B (Fig. 6B) (patients 1, 2, 8 and 14) contains tumors without a lipogenic profile
Patient 12 showed little change in PL profiles between tumor and normal tissue and can not be classified within cluster A or B.

### References

Beckers, A., Organe, S., Timmermans, L., Scheys, K., Peeters, A., Brusselmans, K., Verhoeven, G., and Swinnen, J. V. (2007). Chemical inhibition of acetyl-CoA carboxylase induces growth arrest and cytotoxicity selectively in cancer cells. Cancer Res 67, 8180-8187.
Bedorf, N., Schomburg, D., Gerth, K., Reichenbach, H., Höfle, G. (1993) Isolation and structure elucidation of soraphen A1, a novel antifungal macrolide from Sorangium cellulosum. Liebigs Ann Chem 1993,1017-21.
Brusselmans, K., De Schrijver, E., Verhoeven, G., and Swinnen, J. V. (2005). RNA interference-mediated silencing of the acetyl-CoA-carboxylase-alpha gene induces growth inhibition and apoptosis of prostate cancer cells. Cancer Res 65, 6719-6725.
Brusselmans, K., and Swinnen, J. V. (2009) The lipogenic switch in Cancer. In Mitochondria and Cancer, KK Singh and L.C. Costello, Eds, Springer, New York, USA pp. 39-59.
Chou, T. C., and Talalay, P. (1984). Quantitative analysis of dose-effect relationships: the combined effects of multiple drugs or enzyme inhibitors. Adv Enzyme Regul 22, 27-55.
Crowe, F., Allen, N., Appleby, P., et al. (2008) Fatty acid composition of plasma phospholipids and risk of prostate cancer in a case-control analysis nested within the European Prospective Investigation into Cancer and Nutrition. Am. J. Clin. Nutrition 88: 1353-1363.
Davies, C. L., Loizidou, M., Cooper, A. J., and Taylor, I. (1999). Effect of gamma-linolenic acid on cellular uptake of structurally related anthracyclines in human drug sensitive and multidrug resistant bladder and breast cancer cell lines. Eur J Cancer 35, 1534-1540. D
e Schrijver, E., Brusselmans, K., Heyns, W., Verhoeven, G., and Swinnen, J. V. (2003). RNA interference-mediated silencing of the fatty acid synthase gene attenuates growth and induces morphological changes and apoptosis of LNCaP prostate cancer cells. Cancer Res 63, 3799-3804.
Dupertuis, Y. M., Meguid, M. M., and Pichard, C. (2007) . Colon cancer therapy: new perspectives of nutritional manipulations using polyunsaturated fatty acids. Curr Opin Clin Nutr Metab Care 10, 427-432.
Fruhwirth, G. O., and Hermetter, A. (2008) Mediation of apoptosis by oxidized phospholipids. In: Lipids in health and medicine. Quinn, P. J. and Wang, X (eds) Springer, pp. 351-367.
Gann, P., Hennekens, C., Sacks, F., et al. (1994) Prospective Study of Plasma Fatty Acids and Risk of Prostate Cancer. J. Natl. Cancer Inst. Feb 16; 86 (4): 281-6.
Gerth, K., Bedorf, N., Irschik, H., Höfle, G., Reichenbach, H. (1994) The soraphens: a family of novel antifungal compounds from Sorangium cellulosum (Myxobacteria). I. Soraphen A1 a: fermentation, isolation, biological properties. J Antibiot (Tokyo) 47, 23-31.
Godley, P., Campbell, M., Gallagher, P., et al. (1996). Biomarkers of Essential Fatty Acid Consumption and Risk of Prostatic Carcinoma. Cancer Epidemiology, Biomarkers & Prevention 5, 889-895.
Harvei, S., Bjerve, K., Tretli, S., et al. (1997). Prediagnostic level of fatty acids in serum phospholipds: omega-3 and omega-6 fatty acids and the risk of prostate cancer. In. J. Cancer 71, 545-551.
Hazen S. L. (2008) Oxidized phospholipids as endogenous pattern recognition ligands in innnate immunity. J Biol Chem 6, 15527-15531.
Le Bivic A., Sari H., Reynier M., Lebec S., Bardin F. (1987) Differences in Lipid Characteristics of Autologous Human Melanoma Cell lines with Distinct Biological Properties. JNC, Vol 79, N° 6
Männistö S., Pietinen, P., Virtanen, M., et al. (2003) Fatty Acids and Risk of Prostate Cancer in a Nested-Control Study in Male Smokers. Cancer Epidemiology, Biomarkers & Prevention 12, 1422-1428.
Menendez, J. A., and Lupu, R (2008). Fatty acid synthase and the lipogenic phenotype in cancer pathogenesis. Nat Rev Cancer 7, 763-777.
Milne, S., Ivanova, P., Forrester, J., and Alex Brown, H. (2006). Lipidomics: an analysis of cellular lipids by ESI-MS. Methods 39, 92-103.
Rabinovich, A. L., and Ripatti, P. O. (1991). On the conformational, physical properties and functions of polyunsaturated acyl chains. Biochim Biophys Acta 1085, 53-62.
Regev, R., Yeheskely-Hayon, D., Katzir, H., and Eytan, G. D. (2005). Transport of anthracyclines and mitoxantrone across membranes by a flip-flop mechanism. Biochem Pharmacol 70, 161-169.
Schneider C., Porter, N. A., Brash, A. R. (2008) Routes to 4-hydroxynonenal: fundamental issues in the mechanisms of lipid peroxidation. J Biol Chem.6, 15539-15543.
Schroeder F., Gardiner JM, 1984 Membrane lipids and enzymes of cultured high-and low-metastatic B16 melanoma variants. Cancer Res 44:3626-3269
Stillwell, W., and Wassall, S. R. (2003). Docosahexaenoic acid: membrane properties of a unique fatty acid. Chem Phys Lipids 126, 1-27.
Swinnen, J. V., Brusselmans, K., and Verhoeven, G. (2006). Increased lipogenesis in cancer cells: new players, novel targets. Curr Opin Clin Nutr Metab Care 9, 358-365.
Tang, D. G., La, E., Kern, J., and Kehrer, J. P. (2002). Fatty acid oxidation and signaling in apoptosis. Biol Chem 383, 425-442.
Thorne, R.F., Meldrum, C.J., Harris, S. J., Dorahy, D. J., Shafren, D. R., Berndt, M. C., Burns, G.F., Bibson, P. G. (1997) CD36 forms covalently associated dimers and multimers in platelets and transfected COS cells. Biochem Biophys Res Commun 240, 812-818.
Van Veldhoven, P. P., and Bell, R. M. (1988). Effect of harvesting methods, growth conditions and growth phase on diacylglycerol levels in cultured human adherent cells. Biochim Biophys Acta 959, 185-196.
Van Veldhoven, P. P., Meyhi, E., and Mannaerts, G. P. (1998). Enzymatic quantitation of cholesterol esters in lipid extracts. Anal Biochem 258, 152-155.
Van Veldhoven, P. P., Swinnen, J. V., Esquenet, M., and Verhoeven, G. (1997). Lipase-based quantitation of triacylglycerols in cellular lipid extracts: requirement for presence of detergent and prior separation by thin-layer chromatography. Lipids 32, 1297-1300.
Waltregny, D., Alami, Y, Clausse, N., de Leval, J., Castronovo, V. (2002).Overexpression of the Homeobox Gene HOXC8 in Human Prostate Cancer Correlates With Loss of Tumor Differentiation. The Prostate 50, 162-169.
West, J. D., Ji, C., Duncan, S. T., Amarnath, V., Schneider, C., Rizzo, C. J., Brash, A. R., and Marnett, L. J. (2004). Induction of apoptosis in colorectal carcinoma cells treated with 4-hydroxy-2-nonenal and structurally related aldehydic products of lipid peroxidation. Chem Res Toxicol 17, 453-462.

## Claims

1. An *in vitro* method for determining the lipogenicity of a tumor in a subject, said method comprising; determining the relative expression level of at least 1 mono-unsaturated phospholipid; and at least 1 poly-unsaturated phospholipid, in tumor sample versus normal; wherein an increase in relative expression level of said mono-unsaturated phospholipids and a decrease in relative expression level of said poly-unsaturated phospholipids is indicative for a more aggressive lipogenic phenotype.

2. An *in vitro* method for determining the lipogenicity of a tumor in a subject, said method comprising; determining the expression level of at least 1 mono-unsaturated phospholipid; and at least 1 poly-unsaturated phospholipid, in tumor sample and normal sample; and wherein an increased ratio of mono-unsaturated versus poly-unsaturated phospholipids in tumor sample compared to normal sample is indicative for a more aggressive lipogenic phenotype.

3. The method according to anyone of claims 1 to 2, wherein the phospholipids are selected from the group comprising; glycerophospholipid, phosphatidic acid, phosphatidylethanolamine, phosphatidylcholine, phosphatidylserine and phosphoinositides.

4. The method according to anyone of claims 1 to 3 wherein the mono-unsaturated phospholipids are the mono-unsaturated phosphatidylcholines (PC) PC34:1; and wherein the poly-unsaturated phospholipids are the poly-unsaturated phosphatidylcholines (PC) PC36:4 and/or PC38:4.

5. The method according to anyone of claims 1 to 4 further comprising; measuring the relative expression or phosphorylation/activation of one or more other biomarkers for an aggressive lipogenic phenotype, in tumor sample versus normal; and wherein an increase in relative expression of said one or more other biomarkers, an increase in relative expression level of said mono-unsaturated phospholipids, and a decrease in relative expression level of said poly-unsaturated phospholipids is indicative for a more aggressive lipogenic phenotype.

6. The method according to claim 5, wherein the one or more other biomarkers for an aggressive lipogenic phenotype are selected from FASN, ACCA, choline kinase and ACLY.

7. The method according to anyone of claims 1 to 6 wherein the tumor sample is an exosome.

8. The method according to anyone of claims 1 to 7, wherein the expression level of phospholipids is determined via the analysis of phospholipids by ESI-MS/MS or other mass spectrometry-based methods including MALDI-TOF and MS-based phospholipids imaging.

9. Use of the prognostic or predictive *in vitro* method as claimed in anyone of claims 1 to 8 for determining the lipogenicity of a tumor in a subject.

## Patentansprüche

1. Ein In-Vitro-Verfahren zur Bestimmung der Lipogenizität eines Tumors in einem Subjekt, wobei das Verfahren folgendes umfasst; das Bestimmen des relativen Expressionsniveaus von mindestens 1 einfach-ungesättigten Phospholipid; und mindestens 1 mehrfach-ungesättigten Phospholipid, bei einer Tumor-Probe im Vergleich zu normal; und wobei eine Erhöhung des relativen Expressionsniveaus des einfach-ungesättigten Phospholipids und eine Abnahme des relativen Expressionsniveaus des mehrfach-ungesättigten Phospholipids einen aggressiveren lipogenen Phänotyp anzeigt.

2. Ein In-Vitro-Verfahren zur Bestimmung der Lipogenizität eines Tumors in einem Subjekt, wobei das Verfahren folgendes umfasst; das Bestimmen des Expressionsniveaus von mindestens 1 einfach-ungesättigten Phospholipid und mindestens 1 mehrfach-ungesättigten Phospholipid, bei einer Tumor-Probe und einer normalen Probe; und wobei ein erhöhtes Verhältnis des einfachungesättigten gegenüber dem mehrfach-ungesättigten Phospholipid in der Tumor-Probe im Vergleich zu einer normalen Probe einen aggressiveren lipogenen Phänotyp anzeigt.

3. Ein Verfahren nach einem der Ansprüche 1 bis 2, wobei die Phospholipide aus der Gruppe der Glycerophospholipide, Phosphatidsäure, Phosphatidylethanolamin, Phosphatidylcholin, Phosphatidylserin und Phosphoinositiden ausgewählt werden.

4. Ein Verfahren nach einem der Ansprüche 1 bis 3, wobei die einfach ungesättigten Phospholipide einfach-ungesättigte Phosphatidylcholine (PC) PC34:1 sind, und wobei die mehrfach-ungesättigten Phospholipide mehrfach-ungesättigte Phosphatidylcholine (PC) PC36:4 und / oder PC38:4 sind.

5. Ein Verfahren nach einem der Ansprüche 1 bis 4, ferner umfassend: das Messen der relativen Expression und Phosphorylierung / Aktivierung eines oder mehrerer anderer Biomarker für einen aggressiven lipogenen Phänotyp, in einer Tumor-Probe im Vergleich zu normal; und wobei ein Anstieg der relativen Expression des besagten oder weiterer, unterschiedlicher Biomarker, ein Anstieg des relativen Expressionsniveaus der einfach-ungesättigten Phospholipiden, und eine Abnahme des relativen Expressionsniveaus besagter mehrfach-ungesättigten Phospholipiden einen aggressiveren lipogenen Phänotyp anzeigt.

6. Ein Verfahren nach Anspruch 5, wobei der eine oder die unterschiedlichen anderen Biomarker für einen aggressiven lipogenen Phänotyp aus FASN, ACCA, Cholin-Kinase und ACLY ausgewählt sind.

7. Ein Verfahren nach einem der Ansprüche 1 bis 6, wobei die Tumor-Probe aus körpereigenen Partikeln besteht.

8. Ein Verfahren nach einem der Ansprüche 1 bis 7, wobei das Expressionsniveau von Phospholipiden über die Analyse von Phospholipiden oder anderen auf ESI-MS/MS-Massenspektrometrie basierten Verfahren wie MALDI-TOF und MS-basierter Phospholipide-Tomographie bestimmt werden.

9. Die Verwendung des prognostischen oder prädikativen In-Vitro-Verfahrens nach einem der Ansprüche 1 bis 8, zur Bestimmung der Lipogenizität eines Tumors in einem Subjekt.

## Revendications

1. Une méthode *in vitro* pour déterminer l'activité de lipogène d'une tumeur chez un sujet, ladite méthode comprenant; la détermination du niveau d'expression relave d'au moins 1 phospholipide mono-insaturé; et au moins 1 phospholipide polyinsaturé, dans un échantillon d'une tumeur par rapport à un échantillon normal; où une augmentation dans le niveau d'expression relative des dits phospholipides mono-insaturés et une réduction dans le niveau d'expression relative des dits phospholipides polyinsaturés indique un phénotype de lipogène plus agressif.

2. Une méthode *in vitro* pour déterminer l'activité lipogène d'une tumeur chez un sujet, de ladite méthode comprenant; la détermination de l'expression du niveau d'au moins 1 phospholipide mono-insaturé ; et au moins 1 phospholipide poly -insaturé, dans un échantillon de tumeur et un échantillon normal; et où un taux augmenté de phospholipides mono-instaurés versus des phospholipides poli-insaturés dans un échantillon de tumeur comparé à un échantillon normal indique un phénotype de lipogène plus agressif.

3. La méthode selon les revendications 1 à 2, où les phospholipides sont sélectionnés dans un groupe comprenant ; du glycérophospholipide, de l'acide phosphatidique, du phosphatidyléthanolamine, de la phosphatidylcholine, de la phosphatidylsérine et des phosphoinositides.

4. La méthode selon les revendications 1 à 3 où les phospholipides mono-insaturés sont les phosphatidylcholines mono-insaturés (PC) PC34:1; et où les phospholipides poly-insaturés sont les phosphatidylcholines poly-insaturés (PC) PC36:4 et/ou PC38:4.

5. La méthode selon les revendications 1 à 4 qui comprennent de plus; la mesure de l'expression relative ou la phosphorylation/activation d'un ou plusieurs biomarqueurs pour un phénotype de lipogène agressif, dans un échantillon de tumeur versus un échantillon normal; et où une augmentation relative de l'expression de celui-ci ou d'autres biomarqueurs, une augmentation dans le niveau d'expression relative des dits phospholipides mono-insaturés, et une réduction dans l'expression relative du niveau des dits phospholipides poly-insaturés indique un phénotype de lipogène plus agressif.

6. La méthode selon la revendication 5, où un ou plusieurs autres biomarqueurs pour un phénotype de lipogène agressif sont sélectionnés dans FASN, ACCA, la choline kinase et ACLY.

7. La méthode selon les revendications 1 à 6 où l'échantillon de la tumeur est un exosome.

8. La méthode selon les revendications 1 à 7, où l'expression du niveau de phospholipides est déterminée à travers l'analyse des phospholipides ESI-MS/MS ou d'autres méthodes basées sur la spectrométrie de masse y compris le MALDI-TOF et l'imagerie des phospholipides basés sur le MS.

9. L'utilisation d'un pronostique ou d'une prédiction d'une méthode *in vitro* comme l'affirment les revendications 1 à 8 pour déterminer l'activité lipogène d'une tumeur chez un sujet.
